# EUROPEAN PATENT SPECIFICATION

(11) **EP 1 807 395 B1**
(45) Date of publication and mention of the grant of the patent: **23.12.2015**
(21) Application number: 05801650.2
(22) Date of filing: 24.10.2005
(51) Int. Cl.: C07D 211/44, C07D 211/94, C07D 251/44, C07D 251/50, C07D 251/54, C08K 5/3435, C08K 5/3492, C09K 15/20

(54) **PROCESS FOR THE SYNTHESIS OF N-ALKOXYAMINES**
VERFAHREN ZUR HERSTELLUNG VON N-ALKOXYLAMINEN
PROCÉDÉ POUR LA PRÉPARATION DE N-ALKOXYLAMINES

(30) Priority: 02.11.2004 EP 04105456
(43) Date of publication of application: 18.07.2007
(60) Divisional application: 15181106.4
(73) Proprietor: BASF SE, 67056 Ludwigshafen (DE)
(72) Inventor: FREY, Markus, CH-4310 Rheinfelden (CH); RAST, Valérie, CH-4056 Basel (CH); MARTINEZ, Francisco, I-43100 Parma (IT); ALVISI, Davide, I-44012 Bondeno (FE) (IT)
(86) International application number: PCT/EP2005/055472
(87) International publication number: WO 2006/048389

(56) References cited:
- EP-A- 0 389 419
- WO-A-03/045919
- WO-A-03/053931
- US-A- 5 844 026
- US-B1- 6 265 473

## Description

The present invention relates to novel processes for the preparation of a sterically hindered amine ether by the transformation of a corresponding oxo-piperidin to a hydroxy or animo substituted sterically hindered amine ether and the preparation of a N-propoxy or N-propenoxy substituted sterically hindered amine and some novel compounds obtainable by these processes. The compounds made by these processes are particularly effective in the stabilization of polymer compositions against harmful effects of light, oxyen and/or heat and as flame-retardants for polymers.

WO 01/92228 describes a process for the preparation of amine ethers, e.g. N-hydrocarbyloxy substituted hindered amine compounds, by the reaction of the corresponding N-oxy intermediate with a hydrocarbon in the presence of an organic hydroperoxide and a copper catalyst.

WO 03/045919 describes a process for the preparation of amine ethers, e.g. N-hydrocarbyloxy substituted hindered amine compounds, by the reaction of the corresponding N-oxy intermediate with a hydrocarbon in the presence of an organic hydroperoxide and an iodide catalyst.

DE19907945A describes the formation of 1-allyloxy substituted sterically hindered amines from 1-allyl substituted sterically hindered amines by oxidation.

WO 98/54174 and US 5,844,026 describe the reductive amination of a N-cyclohexyloxy-2,2,6,6-tetramethyl-4-oxo-piperidine to the corresponding amine.

A problem of the state of the art processes is that undesirable side products are obtained that are hard to remove from the desired products as amine oxides do not react selectively with saturated hydrocarbons. The processes of the present invention avoid this problem as hydrocarbons with unsaturated carbon-carbon bonds react more selectively than saturated hydrocarbons, i.e. compounds prepared according to the instant processes may be purer. The transformation product of the process of the present invention may easily be purified by standard methods such as distillation. The hydrogenation of the unsaturated carbon-carbon bond and the reduction or reductive amination of the carbonyl group in one reation step may save one reaction step and may need less solvents and reagents than the state of the art, i.e. this reaction preformed in two separate reaction steps.

The present invention relates to a process for the preparation of a sterically hindered amine ether of the formula (100) 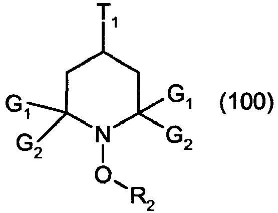 wherein
G₁ and G₂ are independently C₁-C₄alkyl;
R₂ is C₃-C₁₈alkyl or C₅-C₁₂cycloalkyl;
T₁ is hydroxy, -NT₂T₃, -OT₂₂, T₂₀ or a group of formula (102);
T₂ is hydrogen, C₅-C₁₂cycloalkyl or R₄₂; or T₂ is R₄₂ substituted by C₁-C₁₈alkoxy, aryl, hydroxy, carboxy, -CO-O-R₄₂, or-O-CO-R4₂;
T₃ is hydrogen, C₅-C₁₂cycloalkyl, R₄₂, aryl, -Q-NHT₂ or -Q-NT₂T₂₁; or T₃ is R₄₂ substituted by C₁-C₁₈alkoxy, aryl, hydroxy, carboxy, -CO-O-R₄₂, or-O-CO-R₄₂; or T₃ is aryl substituted by C₁-C₁₈alkoxy, aryl, hydroxy, carboxy, -CO-O-R₄₂, -O-CO-R₄₂ or halogen;
or T₂ and T₃ form together C₄-C₁₁alkylene or C₄-C₁₁alkylene substituted by C₁-C₁₈alkoxy, aryl, hydroxy, carboxy, -CO-O-R₄₂, or -O-CO-R₄₂;
with the proviso that T₂ and T₃ are not benzyl;
R₄₂ is C₁-C₁₈alkyl;
Q is C₂-C₁₈alkylene, C₅-C₁₂cycloalkylene or phenylene;
T₂₂ is -(CO)-(C₁-C₁₆alkylene)₀ or ₁-(CO)-O-T₂₁;
T₂₁ is 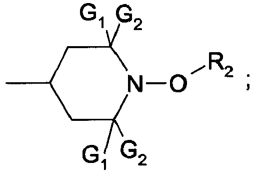
T₂₀ is 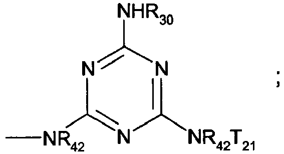
R₃₀ is R₄₂ or R₄₂ substituted by hydroxy; or R₃₀ is -(CH₂)ₙ-NT₂₃-(CH₂)ₚ-NT₂₃-(CH₂)ₙ-NHT₂₃ with one T₂₃ substituent being hydrogen and two T₂₃ substituents being 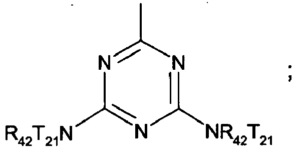
n is 1 to 4;
p is 1 to 3;
the group of formula (102) is 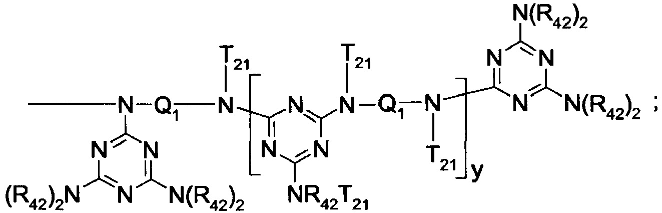
y is 2 to 20;
which comprises transforming a compound of formula (101), 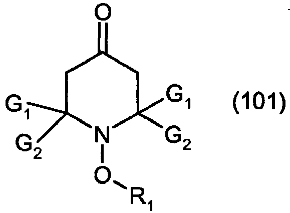 wherein
R₁ is C₃-C₁₈alkenyl or C₅-C₁₂cycloalkenyl,

in one reaction step in the presence of hydrogen and a catalyst into a compound of formula (100) wherein T₁ is hydroxy or -NT₂T₃;
whereby for obtaining compounds with T₁ = -NT₂T₃ the transformation is performed in the presence of an amine of formula HNT₂T₃₀;
T₃₀ is hydrogen, C₅-C₁₂cycloalkyl, R₄₂, aryl or -Q-NHT₂; or T₃₀ is R₄₂ substituted by C₁-C₁₈alkoxy, aryl, hydroxy, carboxy, -CO-O-R₄₂, or -O-CO-R₄₂; or T₃₀ is aryl substituted by C₁-C₁₈alkoxy, aryl, hydroxy, carboxy, -CO-O-R₄₂, -O-CO-R₄₂ or halogen;
or T₂ and T₃₀ form together C₄-C₁₁alkylene or C₄-C₁₁alkylene substituted by C₁-C₁₈alkoxy, aryl, hydroxy, carboxy, -CO-O-R₄₂, or -O-CO-R₄₂;
with the proviso that T₃₀ is not benzyl;
and for obtaining a compound of formula (100) with T₁ = -OT₂₂, reacting a compound of formula (100) with T₁ = hydroxy with an HOOC-(C₁-C₁₆alkylene)₀ or ₁-COOH or a halide thereof or a methyl ester thereof;
for obtaining a compound of formula (100) with T₁ = T₂₀, and R₃₀ = R₄₂ or R₄₂ substituted by hydroxy, reacting a compound of formula (100) with T₁ = -NT₂T₃, T₂ = H, T₃ = R₄₂ with a cyanuric halide to yield a compound of formula (103) [step a1], which is subsequently reacted with R₄₂NH₂ or hydroxy-substituted R₄₂NH₂ [step a2]; 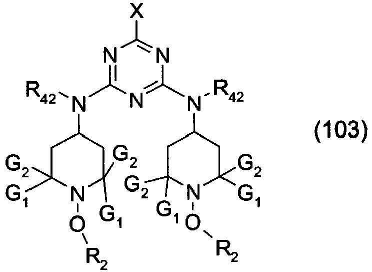 wherein
X is halogen;
for obtaining a compound of formula (100) with T₁ = T₂₀ and R₃₀ = -(CH₂)ₙ-NT₂₃-(CH₂)ₚ-NT₂₃-(CH₂)ₙ-NHT₂₃,
a compound of formula (103) is reacted with H₂N-(CH₂)ₙ-NH-(CH₂)ₚ-NH-(CH₂)ₙ-NH₂; and
for obtaining a compound of formula (100) with T₁ = group of formula (102), reacting a compound of formula (100) with T₁ = -NT₂T₃, T₂ = H, T₃ = R₄₂, with a cyanuric halide to yield a compound of formula (104) [step b1],
which is subsequently reacted with a compound of formula (100) with T₁ = -NT₂T₃, T₂ = H, T₃ = -Q-NHT₂₁, to yield a compound of formula (105) [step b2],
which is subsequently reacted with a compound of formula (100) with T₁ = -NT₂T₃, T₂ = H, T₃ = -Q-NHT₂₁, to yield a compound of formula (106) [step b3],
which is subsequently reacted with a compound 2-X-4,6-bis((R₄₂)₂amino)-s-triazine [step b4].

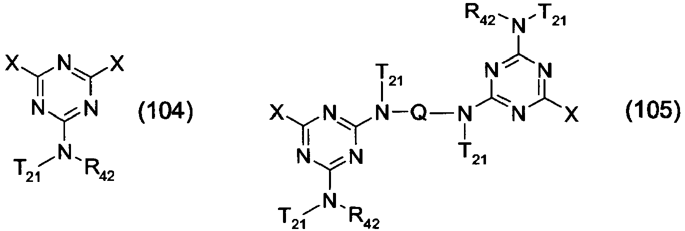

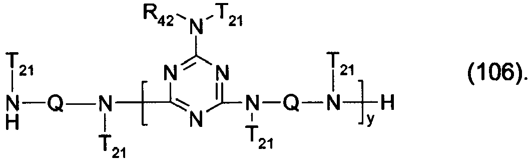

Unless otherwise stated, the reactions described herein are conveniently carried out close to ambient pressure, e.g. between 0.5 and 1.5 bar, especially at about ambient pressure.

The catalyst for the transformation to a compound of formula (100) with T₁ = hydroxy or - NT₂T₃ is preferably Rh, Ir, Ru, Pt or Pd on charcoal or Raney-Ni or a reducing agent such as borohydride. 0.0001-0.1 eq., preferably 0.0005-0.01 eq., especially 0.0005-0.005 eq. catalyst is used in this reaction (eq. are given in molar eq. of the compound of formula 101).

The transformation is preferably carried out at a temperature of 35 - 120°C and a hydrogen pressure of 6 - 100 bar, for example at a temperature of 45 - 110°C and a hydrogen pressure of 8 - 60 bar; also of interest is a temperature of 45 - 110° and a hydrogen pressure of 40 - 60 bar.

The transformation may be carried out in a solvent, preferably an organic solvent or HNT₂T₃₀, for example HNT₂T₃₀, methanol, ethanol, THF, propanol, i-propanol, butanol, 2-butanol, i-butanol, t-butylmethylether, 1,2-dimethoxyethane, dioxane, di-i-propylether, cyclohexane, hexane or heptane.

A compound of formula (100) with T₁ = -OT₂₂ may be obtained by reacting a compound of formula (100) with T₁ = hydroxy with an HOOC-(C₁-C₁₆alkylene)₀ or ₁-COOH or a halide thereof or a methyl ester thereof; such reactions are for example described in C. Ferri, Reaktionen der organischen Synthese, Stuttgart 1978, Georg Thieme Verlag, in particular p. 204 and 447-450 or in R. Larock, comprehensive organic transformations, New York 1989, VCH Verlag, p. 985-987 and the literature cited therein.

The reaction with the halide may be carried out in a neutral, acidic or basic medium, for example in a basic medium such as diluted NaOH, preferably in excess.

The carbonic acid may be reacted in the presence of a catalyst such as inorganic acids, trifluoroacetic acid, arenesulfonacid, ZnCl₂, acidic cation exchanger, SnCl₂ or 2-halogen-1-methylpyridinum salts. The obtained water or diester may be removed from the reaction mixture by distillation. The reaction may be carried out in the absence of a catalyst; in such a case, the reaction may be carried out in the presence of a carbodiimide such as dicyclohexylcarbodiimide.

The reaction with the methyl ester may be carried out in the presence of a catalyst, e.g. NaOAc NaCN, acidic catalyst, (n-C₄H₉)₃SnOR or Ti-, Zr- or Al-alkoxides. Of interest is this reaction being carried out at elevated temperature, for example 50-200° or 50° to the boiling point of the mixture. The compound of formula (100) with T₁ = hydroxy may be used in equimolar amount or in excess, for example 2-10, preferably 3-5 molar equivalents.

The catalyst may be used in 0.5 - 0.01 molar eq., preferably 0.25 - 0.1 molar eq. (eq. are given in molar eq. of the compound of formula (100) with T₁ = hydroxy).

A compound of formula (100) with T₁ = T₂₀, and R₃₀ = R₄₂ or R₄₂ substituted by hydroxy is obtained by reacting a compound of formula (100) with T₁ = -NT₂T₃, T₂ = H, T₃ = R₄₂ with a cyanuric halide to yield a compound of formula (103) [step a1], which is subsequently reacted with R₄₂NH₂ or hydroxy-substituted R₄₂NH₂ [step a2].
Step a1 (as for example described in EP455588; eq. are given as molar eq. of the compound of formula (100) with T₁ = -NT₂T₃, T₂ = H, T₃ = R₄₂):
   The cyanuric halide may be a cyanuric chloride (e.g. 0.1-1 eq., especially 0.4-0.6 eq.). The reaction may be carried out in an organic solvent such as xylene, toluene or cyclohexane in the presence of a base such as NaOH, KOH, NaHCO₃ or Na₂CO₃ in for instance 0.5-1.5 eq., especially 0.9-1.1 eq. and optionally a phase-transfer catalyst such as Bu₄NHSO₄ in 0.0001-0.1 eq., for example 0.001-0.01 eq. The reaction temperature may be 60 - 80°C.
Step a2 (as for instance described in US 5,216,156; eq. are given as molar eq. of the product of step a1):
   R₄₂NH₂ or hydroxy-substituted R₄₂NH₂ may be used in 0.5 - 5 eq. The reaction may be carried out in an organic solvent such as xylene, or in a mixture of xylene and toluene or cyclohexane. Optionally the reaction is carried out in the presence of a base such as NaOH, KOH, NaHCO₃ or Na₂CO₃ in for instance 0.1-1 eq., especially 0.4-0.6 eq. and/or a phase-transfer catalyst such as Bu₄NHSO₄ in 0.0001-0.1 eq, for example 0.001-0.01 eq. The reaction temperature may be 100-130°.

A compound of formula (100) with T₁ = T₂₀ and R₃₀ = -(CH₂)ₙ-NT₂₃-(CH₂)ₚ-NT₂₃-(CH₂)ₙ-NHT₂₃ may be prepared, as described in EP889085A, by reacting two to four equivalents of a compound of formula (103) with one equivalent of H₂N-(CH₂)ₙ-NH-(CH₂)ₚ-NH-(CH₂)ₙ-NH₂; for example the reaction is carried out in a hydrocarbon solvent with an acid acceptor, such as aqueous sodium hydroxide, to neutralize the hydrochloric acid produced in the reaction.

For example, 2.5 to three equivalents of the compound of formula (103), especially three equivalents of the compound of formula (103), are reacted with one equivalent of H₂N-(CH₂)ₙ-NH-(CH₂)ₚ-NH-(CH₂)ₙ-NH₂. This reaction may be carried out in xylene or in a mixture of xylene and toluene or cyclohexane. A base such as NaOH, KOH, NaHCO₃ or Na₂CO₃ (e.g. 2-4 eq.) and optionally a phase-transfer catalyst (e.g. Bu₄NHSO₄ in for instance 0.02-0.04 eq.) may be used in this reaction (eq. are given as molar eq. of H₂N-(CH₂)ₙ-NH-(CH₂)ₚ-NH-(CH₂)ₙ-NH₂). The reaction temperature may be 100-200° C. The reaction may be carried out at a pressure of 0.5-20 bar, for example 0.5-10 bar, especially 0.5-5 bar, for instance at about ambient pressure.

For obtaining a compound of formula (100) with T₁ = group of formula (102), all steps of this reaction may be carried out as for example described in DE19907945.
Step b1 (eq. are given as molar eq. of the compound of formula (100) with T₁ = -NT₂T₃, T₂ = H, T₃ = R₄₂):
   A cyanuric halide such as cyanuric chloride may be used in 0.5-1.5 eq., especially 0.9-1.1 eq. Examples for suitable solvents are xylene, toluene or cyclohexane. A base such as NaOH, KOH, NaHCO₃ or Na₂CO₃ in for instance 0.5-1.5 eq., especially 0.9-1.1 eq. and optionally a phase-transfer catalyst such as Bu₄NHSO₄ in 0.001-0.1 eq, for example 0.005-0.05 eq. may be present in this reaction step. The reaction temperature may be 0 - 40°.
Step b2 (eq. are given as molar eq. of the product of step b1):
   The product of step b1, a compound of formula (100) with T₁ = -NT₂T₃, T₂ = H, T₃ = -Q-NHT₂₁ in 0.1-1 eq., especially 0.4-0.6 eq. and a base such as NaOH, KOH, NaHCO₃ or Na₂CO₃ in for instance 0.5-1.5 eq., especially 0.9-1.1 eq. may be reacted at a temperature of 60 - 80°.
Step b3 (eq. are given as molar eq. of the product of step b2):
   The product of step b2 may be reacted with a compound of formula (100) with T₁ = -NT₂T₃, T₂ = H, T₃ = -Q-NHT₂₁ (e.g. 0.5-1.5 eq., especially 0.9-1.1 eq.) and optionally a base such as NaOH, KOH, NaHCO₃ or Na₂CO₃ (for instance 0.5-1.5 eq., especially 0.9-1.1 eq.) at a reaction temperature of for instance 100-200°.
Step b4 (eq. are given as molar eq. of the product of step b3):
   The product of step b3 is reacted with 2-X-4,6-bis((R42)2amino-s-triazine (e.g. 0.1-1 eq., especially 0.4-0.6 eq.) optionally in the presence of a base such as NaOH, KOH, NaHCO₃ or Na₂CO₃ (for instance 0.1-1 eq., especially 0.4-0.6 eq.), at a reaction temperature of for example 100-200°.

The steps b3 and b4 may be carried out at a pressure of 0.5-20 bar, for example 0.5-10 bar, especially 0.5-5 bar, for instance at about ambient pressure.

Of interest is a process, wherein
R₂ is C₃-C₁₀alkyl or C₅-C₇cycloalkyl;
T₂ is hydrogen;
T₃ is R₄₂, -Q-NHT₂ or -Q-NT₂T₂₁;
R₄₂ is C₁-C₈alkyl;
Q is C₂-C₈alkylene;
T₂₂ is -(CO)-C₄-C₁₀alkylene-(CO)-O-T₂₁;
n is 2 to 4;
y is 2 to 10
R₁ is C₃-C₁₀alkenyl or C₅-C₇cycloalkenyl and
X is chlorine, bromine or iodine.

For example, X is chlorine.

Of technical interest is R₂ being C₃ or C₈alkyl or C₆cyclohexyl and R₁ being C₃ or C₈alkenyl or C₆cyclohexenyl.

Of interest is R₂ being C₃alkyl, R₁ being C₃alkenyl and T₁ being -NT₂T₃.

An embodiment of the present invention is a process, wherein the compound of formula (101) is obtained by reacting a compound of formula (200) with a C₃-C₁₈alkene or C₅-C₁₂cycloalkene.

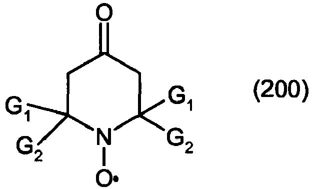

The C₃-C₁₈alkene may be an unbranched alkene, for example a C₃-C₁₈alk-1-ene. Of interest are a C₃-C₁₀alkene or a C₅-C₇alkene, for example C₃ or C₈alkene or C₆cyclohexane, especially C₃alkene.

This process is preferably carried out in the presence of an organic hydroperoxide and optionally a further catalyst.

The further catalyst is preferably selected from the group consisting of scandium, titanium, vanadium, chromium, manganese, iron, cobalt, nickel, copper, zinc, gallium, germanium, yttrium, zirconium, niobium, molybdenum, ruthenium, rhodium, palladium, silver, cadmium, indium, tin, antimony, lanthanum, cerium, hafnium, tantalum, tungsten, rhenium, osmium, iridium, platinum, gold, mercury, thallium, lead, bismuth; the compounds thereof; substituted and unsubstituted ammonium iodides and phosphonium iodides.

The further catalyst may also be quaternary ammonium or phosphonium halogenides such as chlorides or bromides. The structure of the ammonium or phosphonium cation is less important; usually, quaternary ammonium or phosphonium cations contain 4 hydrocarbon residues bonded to the central nitrogen or phosphorus atom, which may be, for example, alkyl, phenylalkyl or phenyl groups. Some readily available materials are tetra-C₁-C₁₂alkylated.

The further catalyst may also be any other iodide compound, including organic and inorganic iodide compounds. Examples are alkaline or alkaline earth metal iodides, or onium iodides such as sulfonium iodides, especially quarternary sulfonium iodides. Suitable metal iodides are, inter alia, those of lithium, sodium, potassium, magnesium or calcium.

The further catalyst is more preferably selected from the group consisting of titanium, vanadium, chromium, manganese, iron, cobalt, nickel, copper, zinc, cerium; the halides and oxides thereof; substituted and unsubstituted ammonium iodides and phosphonium iodides.

The further catalyst is most preferably selected from the group consisting of manganese, iron, cobalt, nickel, copper; the halides thereof; substituted and unsubstituted ammonium iodides and phosphonium iodides, for example substituted and unsubstituted quaternary ammonium or phosphonium iodides, especially tetraalkyl ammonium iodides or tetraphenylphosphonium iodide and triphenylalkylphosphonium iodides.

The further catalyst can be bound to an organic or inorganic polymer backbone, rendering a homogenous or heterogeneous catalytic system.

The further catalysts mentioned above may contain anionic ligands commonly known in complex chemistry of transition metals, such hydride ions (H⁻) or anions derived from inorganic or organic acids, examples being halides, e.g. F⁻, Cl⁻, Br⁻ or I⁻, fluoro complexes of the type BF₄⁻, PF₆⁻, SbF₆⁻ or AsF₆⁻, anions of oxygen acids, alcoholates or acetylides or anions of cyclopentadiene or oxides.

Anions of oxygen acids are, for example, sulfate, phosphate, perchlorate, perbromate, periodate, antimonate, arsenate, nitrate, carbonate, the anion of a C₁-C₈carboxylic acid, such as formate, acetate, propionate, butyrate, benzoate, phenylacetate, mono-, di- or trichloro- or -fluoroacetate, sulfonates, for example methylsulfonate, ethylsulfonate, propylsulfonate, butylsulfonate, trifluoromethylsulfonate (triflate), unsubstituted or C₁-C₄alkyl-, C₁-C₄alkoxy- or halo-, especially fluoro-, chloro- or bromo-substituted phenylsulfonate or benzylsulfonate, for example tosylate, mesylate, brosylate, p-methoxy- or p-ethoxyphenylsulfonate, pentafluorophenylsulfonate or 2,4,6-triisopropylsulfonate, phosphonates, for example methylphosphonate, ethylphosphonate, propylphosphonate, butylphosphonate, phenylphosphonate, p-methylphenylphosphonate or benzylphosphonate, carboxylates derived from a C₁-C₈carboxylic acid, for example formate, acetate, propionate, butyrate, benzoate, phenylacetate, mono-, di- or trichloro- or -fluoroacetate, and also C₁-C₁₂-alcoholates, such as straight chain or branched C₁-C₁₂-alcoholates, e.g. methanolate or ethanolate. Also oxides are possible.

Anionic and neutral ligands may also be present up to the preferred coordination number of the complex cation of the further catalyst, especially four, five or six. Additional negative charges are counterbalanced by cations, especially monovalent cations such as Na⁺, K⁺, NH₄⁺ or (C₁-C₄ alkyl)₄N⁺.

The further catalysts mentioned above may also contain neutral ligands such as inorganic or organic neutral ligands commonly known in complex chemistry of transition metals. Suitable inorganic ligands are selected from the group consisting of aquo (H₂O), amino, nitrogen, carbon monoxide and nitrosyl. Suitable organic ligands are selected from the group consisting of phosphines, e.g. (C₆H₅)₃P, (i-C₃H₇)₃P, (C₅H₉)₃P or (C₆H₁₁)₃P, di-, tri-, tetra- and hydroxyamines, such as ethylenediamine, ethylenediaminotetraacetate (EDTA), N,N-dimethyl-N',N'-bis(2-dimethylaminoethyl)-ethylenediamine (Me₆TREN), catechol, N,N'-dimethyl-1,2-benzenediamine, 2-(methylamino)phenol, 3-(methylamino)-2-butanol or N,N'-bis(1,1-dimethylethyl)-1,2-ethanediamine, N,N,N',N",N"-pentamethyldiethyltriamine (PMDETA), C₁-C₈-glycols or glycerides, e.g. ethylene or propylene glycol or derivatives thereof, e.g. di-, tri- or tetraglyme, and monodentate or bidentate heterocyclic e⁻ donor ligands.

The further catalyst can further contain heterocyclic e⁻ donor ligands which are derived, for example, from unsubstituted or substituted heteroarenes from the group consisting of furan, thiophene, pyrrole, pyridine, bis-pyridine, picolylimine, g-pyran, g-thiopyran, phenanthroline, pyrimidine, bis-pyrimidine, pyrazine, indole, coumarone, thionaphthene, carbazole, dibenzofuran, dibenzothiophene, pyrazole, imidazole, benzimidazole, oxazole, thiazole, bis-thiazole, isoxazole, isothiazole, quinoline, bis-quinoline, isoquinoline, bis-isoquinoline, acridine, chromene, phenazine, phenoxazine, phenothiazine, triazine, thianthrene, purine, bis-imidazole and bis-oxazole.

The sterically hindered aminoxides, also referred to as N-oxyl educts for the instant process which include compounds with at least one group of formula (200), are largely known in the art; they may be prepared by oxidation of the corresponding N-H hindered amine with a suitable oxygen donor, e.g. by the reaction of the corresponding N-H hindered amine with hydrogen peroxide and sodium tungstate as described by E. G. Rozantsev et al., in Synthesis, 1971, 192; or with tert-butyl hydroperoxide and molybdenum (VI) as taught in United States Patent No. 4,691,015, or obtained in analogous manner.

The amount of C₃-C₁₈alkene or C₅-C₁₂cycloalkene is typically a ratio of 1 to 100 moles of C₅-C₁₈alk-1-ene per mole of compound of formula (200) with the preferred ratio being 1 to 50 moles per mole of compound of formula (200), and the most preferred ratio being 1 to 30 moles of C₁₅-C₁₈alk-1-ene per mole of compound of formula (200).

For example, the amount of organic hydroperoxide is 0.5 to 20 moles per mole of compound of formula (200), with the preferred amount being 0.5 to 5 moles of peroxide per mole of compound of formula (200) and the most preferred amount being 0.5 to 3 moles of peroxide per mole of compound of formula (200).

The organic hydroperoxide used in the process of present invention can be of the formula R-OOH, wherein R usually is a hydrocarbon containing 1-18, preferably 3-18 carbon atoms. R is advantageously aliphatic, for example an alkyl group, preferably C₁-C₁₂alkyl. Most preferably, the organic hydroperoxide is tert-butyl-hydroperoxide or cumyl hydroperoxide.

The preferred amount of further catalyst is from about 0.0001 to 0.5, especially 0.0005 to 0.1 molar equivalent per mole of compound of formula (200), with a ratio of 0.001 to 0.05 moles of further catalyst per mole of compound of formula (200) being the most preferred.

The reaction is preferably run at 0° to 100°C; more preferably at 20° to 100°C, especially in the range from 20 to 80°C.

The C₅-C₁₈alkene or C₅-C₁₂cycloalkene may serve two functions both as reactant and as solvent for the reaction. The reaction can also be carried out using an inert organic or inorganic solvent.

Such solvent may be used, especially if the further catalyst is not very soluble in the C₅-C₁₈alk-1-ene. Typical inert solvents are acetonitrile, aromatic hydrocarbons like benzene, chlorobenzene, CCl₄, alcohols (e.g. methanol, ethanol, ethylene glycol, ethylene glycol monomethyl ether), or alkanes like hexane, decane etc., or mixtures thereof. Inorganic solvents such as water are possible as well.

The instant process can be run in air or in an inert atmosphere such as nitrogen or argon. The instant process can be run under ambient pressure as well as under reduced or elevated pressure.

There are several variations of the instant process. One variation involves the addition of a solution of organic hydroperoxide to a mixture of the N-oxyl hindered amine, the C₅-C₁₈alkene or C₅-C₁₂cycloalkene and solvent (if used), and optionally further catalyst which has been brought to the desired temperature for reaction. The proper temperature may be maintained by controlling the rate of peroxide addition and/or by using a heating or cooling bath. After the hydroperoxide is added, the reaction mixture is conveniently stirred till the starting amineoxide has disappeared or is no longer being converted to the desired product, e.g. compound of formula (101). The reaction can be monitored by methods known in the art such as UV-VIS spectroscopy, thin layer chromatography, gas chromatography or liquid chromatography. Additional portions of catalyst can be added while the reaction is in progress. After the initial hydroperoxide charge has been added to the reaction mixture, more hydroperoxide can be added dropwise to bring the reaction to completion.

A second variation of the instant process is to simultaneously add separate solutions of the hydroperoxide and the compound of formula (200) to a mixture of the C₅-C₁₈alkene or C₅-C₁₂cycloalkene, solvent (if used) and optionally further catalyst. The compound of formula (200) may be dissolved in water or the solvent used in the reaction, for example an alcohol. Some of the compound of formula (200) may be introduced into the reaction mixture prior to starting the peroxide addition, and all of the compound of formula (200) should be added prior to completing the peroxide addition.

Another variation of the instant process involves the simultaneous addition of separate solutions of the hydroperoxide and of the aqueous or solvent solution of the further catalyst to a mixture of the compound of formula (200), C₅-C₁₈alk-1-ene or C₅-C₁₂cycloalkene, and solvent (if used). Some of the further catalyst may be introduced into the reaction mixture prior to starting the peroxide addition.

Still another variation of the instant process is the simultaneous addition of separate solutions of the hydroperoxide, of the aqueous or solvent solution of the nitroxyl compound, and of an aqueous or solvent solution of the further catalyst to the C₅-C₁₈alk-1-ene or C₅-C₁₂cycloalkene and solvent (if used). A portion of the compound of formula (200) and/or catalyst may be introduced into the reaction mixture prior to starting the hydroperoxide addition. All of the compound of formula (200) should be added prior to completing the hydroperoxide addition.

At the end of the reaction, the residual hydroperoxide may be carefully decomposed prior to the isolation of any products.

Another embodiment of the present invention is a process, wherein the compound of formula (200) is obtained by oxidizing a compound of formula (201).

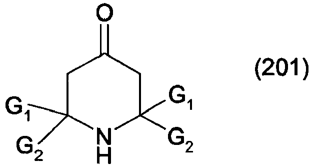

The sterically hindered aminoxides, which include compounds of formula (200), are largely known in the art; they may be prepared by oxidation of the corresponding N-H hindered amine with a suitable oxygen donor, e.g. by the reaction of the corresponding N-H hindered amine with hydrogen peroxide and sodium tungstate as described by E. G. Rozantsev et al., in Synthesis, 1971, 192; or with tert-butyl hydroperoxide and molybdenum (VI) as taught in United States Patent No. 4,691,015, or obtained in analogous manner. Starting compounds of formula (201) are known in the art, are partly commercially available or can be synthesised according to procedures known in the art as for example described in US 4,734,502.

The above-mentioned processes may comprise the conversion of a compound of formula (201) to a compound of formula (100) without the isolation of the intermediate products.

For instance, the above-mentioned processes may comprises the conversion of a compound of formula (200) to a compound of formula (100) without the isolation of the intermediate products.

The compound of formula (101) with R₁ being the group 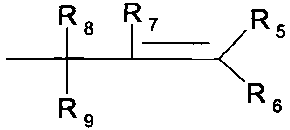 wherein
R₅, R₆, R₇, R₈ and R₈, independently of each other, are H, C₁-C₈alkyl, C₂-C₈alkenyl; and R₇ and R₈ together may also form a chemical bond;
is obtained by a process involving the following: reacting a compound of formula (202) with a compound of formula (203), 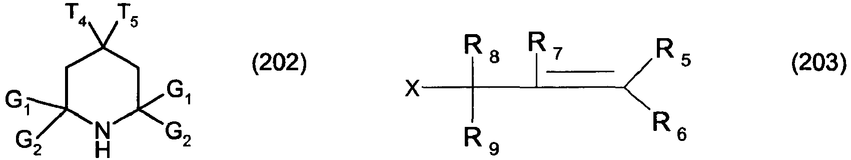 wherein T₄ and T₅ are independently C₁-C₁₈alkoxy; or T₄ is hydroxy and T₅ is hydrogen; X is halogen;
affording a compound of formula (204); 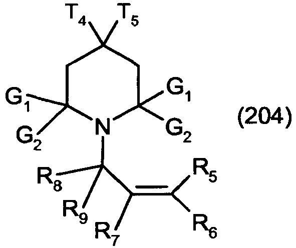 oxidizing the compound of formula (204) in the presence of oxygen, peroxides, permanganates or chlorates affords a compound of formula (205); and 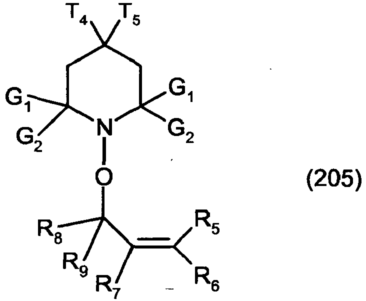 deacetalising the compound of formula (205) with T₄ and T₅ being independently C₁-C₁₈alkoxy or oxidizing the compound of formula (205) with T₄ = hydroxy and T₅ = hydrogen.

Starting compounds of (202) are known in the art, are partly commercially available or can be synthesised according to procedures known in the art as for example described in EP0748849 A.

The compound of formula (204) may be obtained from the compounds of formulae (202) and (203) as described in C. Ferri, Reaktionen der organischen Synthese, Stuttgart 1978, Georg Thieme Verlag, in particular p. 211-212 and the literature cited therein. The molar ratio of the compound of formula (202) to the compound of formula (203) is 0.5 to 4, preferably 1 to 3, most preferably 1.5 to 2.5. As catalyst, Cu or Pd powder, Cu or Pd salt or phosphine complexes thereof or quarternary ammonium salt such as Bu₄N⁺ salts, for example Bu₄NHSO₄ may be used in catalytic amounts. The reaction may be carried out with or without a solvent. Suitable solvents can be hydrocarbons (e.g. xylene or toluene), alcohols (especially methanol or ethanol), ethers (e.g. tetrahydrofuran) or molar solvents like dimethylformamide or N-methyl-2-pyrrolidone. The reaction temperature may be 20 - 150°, for example 50 - 120° or for reactions including a solvent 50° to the boiling point of the solvent. Optionally, a base such as an alkali metal carbonate, hydrogencarbonate or hydroxide, for example Na₂CO₃, NaHCO₃ or NaOH, may be present as a reagent.

X in formula (203) is preferably chlorine, bromine or iodine, most preferably bromine or iodine.

The oxidation to obtain the compound of formula (205) from the compound of formula (204) can be carried out using known oxidants, e.g. oxygen, peroxides or other oxidizing agents such as nitrates, permanganates, chlorates; preferred are peroxides, such as hydrogen peroxide based systems, especially peracids such as perbenzoic acid or peracetic acid. The oxidant is conveniently used in stoichiometric amount or in excess, e.g. using 1-2 moles active oxygen atoms for each compound of formula (204).

The reaction can be carried out in the presence of a suitable solvent, for example an aromatic or aliphatic hydrocarbon, alcohol, ester, amide, ether, or halogenated hydrocarbon; examples are benzene, toluene, xylene, mesitylene, methanol, ethanol, propanol, butanol, dimethylformamide, dimethylsulfoxide, methylene chloride; preferred is a C₁-C₄alcohol, benzene, toluene, xylene, or chlorinated C₁-C₆hydrocarbon.

Temperature and pressure are not critical and depend mainly on the oxidant system used; preferably, temperature is kept during the reaction in the range between -20°C and +40°C. Conveniently, the pressure is kept close to ambient pressure, e.g. between 0.5 and 1.5 bar; when oxidation is achieved with gaseous oxygen, the pressure of oxygen or oxygen/inertgas may exceed ambient pressure.

Deacetalising the compound of formula (205) with T₄ and T₅ being independently C₁-C₁₈alkoxy may be carried out by known methods as for example described in C. Ferri, Reaktionen der organischen Synthese, Stuttgart 1978, Georg Thieme Verlag, particularly p.241 or J. March, Advanced organic chemistry, 3. edition, New York 1985, Wiley-Interscience, in particular p. 329-331 or in Th. Greene, protective groups in organic synthesis, John Wiley & Sons Inc., New York 1991, p. 180-183 and the literature cited in these references. The deacetalising may be carried out in an organic solvent as for example tetrahydrofuran in the presence of water and an acid. The acid may be HCl, HBr or HI, especially HCl. Water may be used in excess, i.e. more than one mol water per mol of compound of formula (205). The deacetalising may be carried out with LiBF₄ in wet acetonitrile or in nonaqueous conditions with Me₃SiI in methylenechloride or in chloroform. Of technical interest is the deacetalisinge using H₂O/HCl. 1-100 eq., preferably 10-50 eq. water, 0.01-10 eq., preferably 0.1-1 eq. HCl and a co-solvent such as THF, MeOH or EtOH is used. The reaction temperature may be 0 - 80°, preferably 20-50°C.

Oxidizing the compound of formula (205) with T₄ = hydroxy and T₅ = hydrogen is carried out by known methods such as described in J. March, Advanced Organic Chemistry, John Wiley & Sons, New York, 1992, p. 1167 - 1171 and the literature cited therein.

Primary oxidants may be, but are not limited to, those being industrially attractive because they are both, cheap and environmentally benign, such as e.g. a catalyst and a further substance selected from the group consisting of oxygen, hydrogenperoxide, a hypochlorite, an alkylhydroperoxide and a carbonyl compound:
a) Oxygen and a catalyst such as a nitroxide (2,2,6,6-tetramethylpiperidine-N-oxide (TEMPO), 4-[C₁-C₁₆alkyl oxy, C₁-C₁₆alkanoyl oxy or aroyl oxy]-TEMPO, Chimassorb® 944 or compound K' of Example 12), N-hydroxyphtalimide, N,N,N-trihydroxyisocyanuric acid or N-hydroxysaccharin together with one or more of the following co-catalysts a polyoxometallic acid or its alkali or tetraalkylammonium salt (e.g. H₅[PMo₁₀V₂O₄₀]; tungstates, phosphotungstates, silicotungstates, borotungstates, vanadates, molybdates, phosphomolybdates, silicomolybdates, titanates or silicotitanates); a group VIIA, VIIIA or IB metal, an oxide thereof, a salt thereof (e.g. chlorides, bromides, acetates or acetylacetonates) or a complex thereof (e.g. Pd[PPh₃]₂Cl₂, Pd[PPh₃]₄, Ru[PPh₃]₄H₂, Ru(PPh₃)₃Cl₂ or Cu[1,10-phenantroline]Cl); enzymes such as chloroperoxidase; further co-catalysts or co-additives may be alkali, earthalkali or tetraalkylammonium iodides; sym-dicarbethoxy hydrazine or diethyl azodicarboxylate; benzoic, 3-chlorobenzoic, phtalic or iso-phtalic acid; alkali hydrogencarbonates or carbonates; hydroquinone or p-benzoquinone; ascorbic acid.
b) Hydrogenperoxide and a catalyst such as a polyoxometallate as described above (e.g. Na₂WO₄) or an enzyme (e.g. chloroperoxidase), together with one or more of the following co-catalysts: a nitroxide as defined above or its deoxygenated precursor (amine); a phase-transfer agent such as tetraalkylammonium halides (especially chlorides, bromides, iodides or hydrogensulfates, e.g. trioctylmethylammonium hydrogensulfate).
c) a hypochlorite and a catalyst such as a nitroxide defined as above together with a cocatalyst such as alkali or earthalkali bromides or iodides or alkali borates.
d) an alkylhydroperoxide (e.g. t-butylhydroperoxide or cumylhydroperoxide) and a catalyst such as Al-, Zr- or Ti-alkoxides (for instance n-propoxides, i-propoxides or t-butoxides, e.g. Zr[OⁿPr]₄, Zr[OⁱPr]₄ or Zr[O^{t}Bu]₄), ZrO(OAc)₂ or an enzyme (e.g. chloroperoxidase).
e) carbonyl compounds such as ketones (e.g. acetone, 2-butanone, 3-pentanone, 4-methyl-2-pentanone, cyclohexanone) and a catalyst such as Al-, Zr- or Ti-alkoxides (e.g. Al[OⁿPr]₃, Al[OⁱPr]₃ or Al[O^{t}Bu]₃, metals (e.g. Pt, Pd, Ru or Raney Nickel) or Ru complexes (e.g. Ru[PPh₃]₄H₂ or Ru(PPh₃)₃Cl₂).

The catalysis may be homogeneous or heterogenous, and the reaction may be homogeneous (one-phase) or heterogeneous (two- or more phases).

In the examples for reactions a) to e), equivalents (eq.) are given as molar equivalents of a compound of formula (205) with T₄ = hydroxy and T₅ = hydrogen unless otherwise stated.

Examples for oxygen and a catalyst are:
a1) described by R. Neumann et al., J. Org. Chem. 66, 8650-8653 (2001):
   0.001-0.1, preferably 0.005-0.05 eq. H₅[PMo₁₀V₂O₄₀]; 0.001-0.1, preferably 0.005-0.05, especially 0.02-0.04 eq. TEMPO; the reaction may be carried out in a solvent such as acetone or a mixture of acetone and 2-butanone, 3-pentanone, 4-methyl-2-pentanone or cyclohexanone; the pressure of oxygen may be 1-10, for example 1.5-5, about 1.5-2.5 atm; the reaction temperature may be 25-125°, preferably 50-110°, especially 90-110°.
a2) described by A. Sheldon et al., J. Am. Chem. Soc. 123, 6826-6833 (2001):
   0.001-0.1, preferably 0.005-0.05 eq. RuCl₂(PPh₃)₃; 0.001-0.2, preferably 0.015-0.15 eq. TEMPO; the reaction may be carried out in a solvent such as chlorobenzene; the pressure of oxygen may be 1-20, preferably 5-15, for example 8-12 bar; the reaction temperature may be 25-125°, preferably 50-110°, especially 90-110°.
a3) described by Y. Ishii et al., J. Org. Chem. 65, 6502-6507 (2000):
   0.01-0.2, preferably 0.05-0.15 eq. N-hydroxyphtalimide; 0.001-0.1, preferably 0.002-0.05 eq. Co(OAc)₂; 0.01-0.5, preferably 0.02-0.1 eq. m-chlorobenzoic acid; the reaction may be carried out in a solvent such as ethylacetate or in a mixture of ethylacetate and chlorobenzene, acetonitrile or methylacetate; the pressure of oxygen may be 0.5-50, preferably 0.5-25, for example 0.5-2 bar; the reaction temperature may be 0-100°, preferably 20-50°, especially 20-30°.
a4) described by I. Marko et al., J. Org. Chem. 64, 2433-2439 (1999):
   0.01-0.5, preferably 0.02-0.1 eq. Cu[1,10-phenantroline]Cl; 0.01-0.5, preferably 0.02-0.1 eq. *sym-*dicarbethoxy hydrazine or diethyl azodicarboxylate; 0.1-4, preferably 1-3 eq. K₂CO₃; the reaction may be carried out in a solvent such as toluene or a mixture of toluene with chlorobenzene, acetonitrile, ethylacetate or methylacetate; the pressure of oxygen may be 0.5-50, preferably 0.5-25, especially 0.5-1.5 bar; the reaction temperature may be 25-120°, preferably 50-100°, especially 80-100°.

An example of hydrogenperoxide and a catalyst is described by R. Noyori et al., Chem. Commun. 2003, 1977-1986:
0.001-0.1, preferably 0.0015-0.05 eq. Na₂WO₄; 0.001-0.1, preferably 0.0015-0.05 eq. trioctylmethylammonium hydrogensulfate; 1-5, preferably 1-2 eq. H₂O₂ (e.g. aqueous 25-35%); the reaction temperature may be 25-100°, preferably 50-100°, especially 85-95°.

An example of hypochlorite and a catalyst is described by H. van Bekkum et al., Synthesis 10, 1153-1174 (1996):
0.001-0.1, preferably 0.005-0.05 eq. 4-methoxy-TEMPO; 0.01-0.3, preferably 0.05-0.2 eq. KBr; 1-3, preferably 1.1-1.75 eq. NaOCl (e.g. 0.35 molar); the reaction may be carried out in a solvent such as dichloromethane or a mixture of dichloromethane and 1,2-dichloroethane, ethylacetate, methylacetate, chlorobenzene or toluene; the reaction temperature may be -10 to 50°, preferably -5 to 30°, especially -5 to 10°.

An example of an alkylhydroperoxide and a catalyst is described by H. Adam et al., J. Org. Chem. 61, 1467-1472 (1996):
0.01-1, preferably 0.05-0.5 eq. Zr(OⁿPr)₄ or Zr(OtBu)₄; 1-5, preferably 1.5-3 eq. t-BuOOH (e.g. anhydrous); the reaction may be carried out in a solvent such as toluene or a mixture of toluene and cyclohexane, hexane, dichloromethane, chloroform, 1,2-dichloroethane, ethylacetate or methylacetate; optionally the reaction is carried out in the presence of molecular sieves; the reaction temperature may be -25 to 100°, preferably 0-80°, especially 20-50°.

Examples for carbonyl compounds and a catalyst are:
e1) described by J. Bäckvall et al., J. Org. Chem. 61, 6587-6590 (1996):
   0.001-0.05, preferably 0.0015-0.03 eq. Ru(PPh₃)₃Cl₂; examples of carbonyl compounds are acetone, 2-butanone, 3-pentanone, 4-methyl-2-pentanone and cyclohexanone, wherein acetone may be used as solvent as well; the reaction temperature may be 25-120°, preferably 50-100°, especially about the reflux temperature of the reaction mixture.
e2) described by Houben-Weyl, Methoden der Organischen Chemie, Georg Thieme Verlag, Stuttgart 1973, vol. 7/2a, p. 714-718 and Org. Synth. IV, 192-195 (1963):
   0.01-0.8, preferably 0.05-0.6 eq., especially 0.4-0.6 eq. Al(OtBu)₃ or Al(OiPr)₃; examples of carbonyl compounds are cyclohexanone, acetone, 2-butanone, 3-pentanone and 4-methyl-2-pentanone, usually 1-50, preferably 10-30 eq. of the carbonyl compound is used; a mixture of toluene and chlorobenzene, THF, 1,4-dioxane or 1,2-dichloroethane may be used as solvent; the reaction temperature may be 25-130°, preferably 50-120°, especially about the reflux temperature.

Preference is given to a reaction carried out in presence of alkylhydroperoxides and a catalyst as described above.

Compounds of formula (205) may also be obtained from a compound of formula (202) in analogous manner as the reaction of compound of formula (201) to a compound of formula (200) with consecutive reaction to a compound of formula (101). So compounds of formula (202) may be oxidized and the obtained product may be reacted with a C₃-C₁₈alkene or C₅-C₁₂cycloalkene as described above. Such a reaction sequence is shown in Example 11.

Compounds of formula (205) may be directly converted to compounds of formula (100) by initial imine formation by subsequent hydrogenation. This reaction may be catalyzed by e.g. Sc(OTf)₃ or by La(OTf)₃. Such a reaction is described for example in H. Heaney et al., Synlett. 1998, 640-642.

In the above-mentioned processes, G₁ and G₂ are for example methyl.

In the definitions the term alkene comprises, for example propene, and the branched and unbranched isomers of butene, pentene, hexene, heptene, octene, nonene, decene, undecene and dodecene. The term alkene also comprises residues with more than one double bond that may be conjugated or non-conjugated, for example may comprise one double bond.

Some examples of cycloalkene are cyclopentene, cyclohexene, methylcyclopentene, dimethylcyclopentene and methylcyclohexene. Cycloalkene may comprise more than one double bond that may be conjugated or non-conjugated, for example may comprise one double bond.

In the definitions the term alkyl comprises within the given limits of carbon atoms, for example methyl, ethyl, propyl, isopropyl, n-butyl, sec-butyl, isobutyl, tert-butyl, 2-ethylbutyl, n-pentyl, isopentyl, 1-methylpentyl, 1,3-dimethylbutyl, n-hexyl, 1-methylhexyl, n-heptyl, 2-methylheptyl, 1,1,3,3-tetramethylbutyl, 1-methylheptyl, 3-methylheptyl, n-octyl, 2-ethylhexyl, 1,1,3-trimethylhexyl, 1,1,3,3-tetramethylpentyl, nonyl, decyl, undecyl, 1-methylundecyl or dodecyl.

Examples of alkenyl are within the given limits of carbon atoms vinyl, allyl, and the branched and unbranched isomers of butenyl, pentenyl, hexenyl, heptenyl, octenyl, nonenyl, decenyl, undecenyl and dodecenyl. The term alkenyl also comprises residues with more than one double bond that may be conjugated or non-conjugated, for example may comprise one double bond.

Examples of alkylene are within the given limits of carbon atoms branched and unbranched isomers of vinylene, allylene, butylene, pentylene, hexylene, heptylene, octylene, nonylene, decylene, undecylene and dodecylene.

Some examples of cycloalkyl are cyclopentyl, cyclohexyl, methylcyclopentyl, dimethylcyclopentyl and methylcyclohexyl.

Some examples of cycloalkenyl are cyclopentenyl, cyclohexenyl, methylcyclopentenyl, dimethylcyclopentenyl and methylcyclohexenyl. Cycloalkenyl may comprise more than one double bond that may be conjugated or non-conjugated, for example may comprise one double bond.

Aryl is for example phenyl or naphthyl.

The term alkoxy may comprise within the limits of the given number of carbon atoms, for example methoxy and ethoxy and the branched and unbranched isomers of propoxy, butoxy, pentyloxy, hexyloxy, heptyloxy, octyloxy, nonyloxy, decyloxy, undecyloxy, dodecyloxy, tridecyloxy, tetradecyloxy, pentadecyloxy, hexadecyloxy, heptadecyloxy and octadecyloxy.

The term halogen may comprises chlorine, bromine and iodine; for example halogen is chlorine except in formula (203).

### Examples

Abbreviations for NOR building blocks:

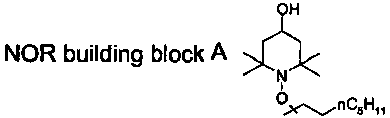

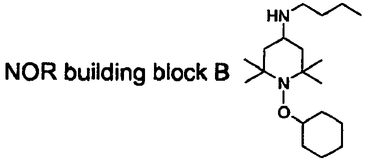

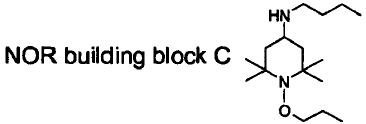

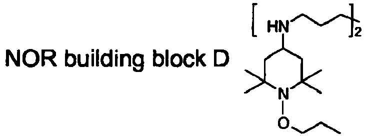

### Example 1: Preparation of NOR building block A in three steps from triacetonamine (TAA)

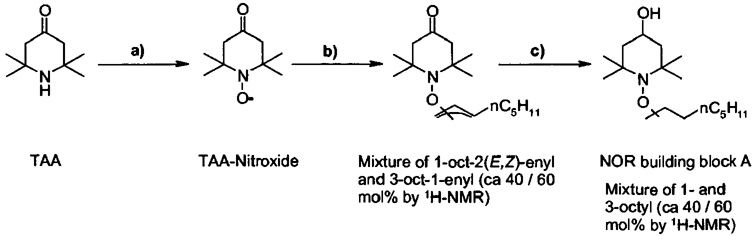

**a)** To a stirred mixture of 41.1 g (0.27 mol) triacetoneamine, 3.94 g (0.01 mol) sodium tungstate dihydrate and 250ml water are added at 5°C within 1 hour 64.9 g (0.57 mol) aqueous 30% hydrogenperoxide. The orange mixture is warmed to 25°C and stirring is continued for 21 hours. Potassium carbonate is then added until phase separation occurs and the triacetoneamine-N-oxide extracted three times with a total of 268 g (2.39 mol) 1-octene.
**b)** After addition of 0.64 g (4.8 mmol) cupric chloride the combined organic phases are brought to 60°C and 39.9 g (0.31 mol) t-butylhydroperoxide slowly dosed in. The mixture is held at 60°C for a total of 5.6 hours. The greenish suspension is then cooled to 25°C followed by the addition of 198 g aqueous 20% sodium sulfite. After stirring overnight the aqueous phase is split off and extracted with hexane. The combined organic phases are washed with water and concentrated on a rotary evaporator.
**c)** The residue is dissolved in 500 ml methanol, 9 g Ru on charcoal (5%) are added and the mixture hydrogenated at 50°C / 45 bar hydrogen during 17 hours. The mixture is filtered through hyflo and the filtrate concentrated on a rotary evaporator. Distillation of the residue yields 40.4 g (53.3%) of a slightly reddish oil (bp 123°C / 0.4 mbar) consisting of a mixture of 2,2,6,6-tetramethyl-1-octyloxy-piperidin-4-ol (ca 40 mol% by ¹H-NMR) and 1-(1-ethyl-hexyloxy)-2,2,6,6-tetramethyl-piperidin-4-ol (ca 60 mol% by ¹H-NMR).

Analysis required for C₁₇H₃₅NO₂ (285.47): C 71.53%, H 12.36%, N 4.91%; found: C 70.62%, H 12.69%, N 4.90%.
¹H-NMR (CDCl₃), δ (ppm, O-C(n)Hₓ only): 3.67 (p-like, O-C(3)H), 3.72 (t, J = ca 6.6 Hz, O-C(1)H₂), 3.95 (broad m, C(4)HOH from heterocycle).
¹³C(DEPT)-NMR (CDCl₃), δ (ppm, O-C(n)Hₓ only): 63.15 and 63.24 (C(4)HOH from heterocycle), 77.01 (O-C(1)H₂), 83.23 (O-C(3)H).

### Example 2: Synthesis of compound A' by transesterification of NOR building block A with sebacic acid dimethylester:

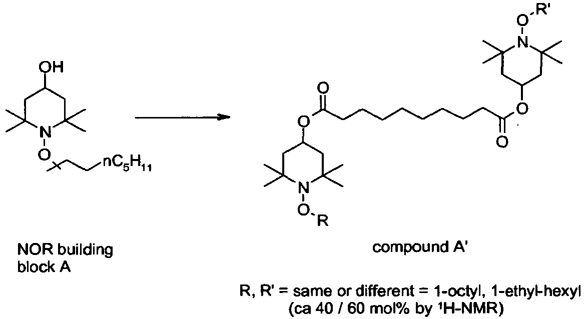

A mixture of 11.4 g (40 mmol) NOR building block A, 1.86 g (8 mmol) sebacic acid dimethylester and 0.135 g (1.6 mmol) LiOtBu are heated under vacuum (110°C, 200 mbar) during 22 hrs. The mixture is diluted with ethylacetate, washed pH neutral and the organic phase concentrated on a rotary evaporator. Flash chromatography (silica gel, hexane / ethylacetate 9 / 1) affords 4 g (68%) of the product as a slightly orange oil.

Analysis required for C₄₄H₈₄N₂O₆ (737.16): C 71.69%, H 11.49%, N 3.80%; found: C 71.47%, H 11.47%, N 3.69%.
¹H-NMR (CDCl₃), δ (ppm, O-C(n)Hₓ only): 3.67 (p-like, O-C(3)H), 3.72 (t, J = 6.8Hz, O-C(1)H₂).
¹³C(DEPT)-NMR (CDCl₃), δ (ppm, O-C(n)Hₓ only): 77.05 (O-C(1)H₂), 83.3 (O-C(3)H).

### Example 3: Preparation of NOR building block B from triacetonamine (TAA):

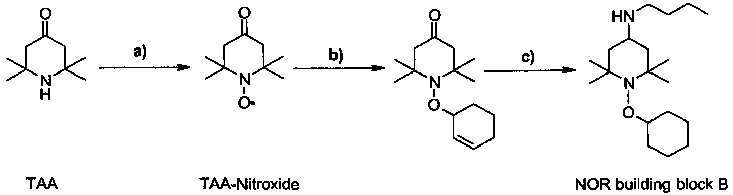

**a)** To a stirred mixture of 76.8g (0.49 mol) triacetoneamine, 7.1g (0.02 mol) sodium tungstate dihydrate and 445ml water are added at 5°C within 1 hour 122.5g (1.08 mol) aqueous 30% hydrogen peroxide. The orange mixture is warmed to 25°C and stirring is continued for 18 hours. Potassium carbonate is then added until phase separation occurs and the triacetoneamine-N-oxide extracted four times with a total of 371.5g (4.52 mol) cyclohexene.
**b)** After addition of 1.01g (4.5 mmol) cupric bromide the combined organic phases are brought to 60°C and 49g (0.38 mol) t-butylhydroperoxide slowly dosed in. The mixture is held at 60°C for a total of 2.3 hours. The greenish suspension is then cooled to 25°C followed by the addition of 280g aqueous 20% sodium sulfite solution. After stirring 1 hour the aqueous phase is split off, the organic phase washed with water and brine and then concentrated on a rotary evaporator.
**c)** The residue is dissolved in 1200ml methanol followed by the addition of 35.8g (0.49 mol) butylamine and 16.3g Pd on charcoal (10%). The mixture is stirred at 25°C during 1.5 hours and then hydrogenated at 50°C / 10 bar hydrogen during 1.5 hours. The mixture is filtered through Hyflo and the filtrate concentrated on a rotary evaporator. Distillation of the residue yields 112g (73%) of a yellow to slightly orange oil (bp 120°C / 0.8 mbar).

Analysis required for C₁₉H₃₈N₂O (310.53): C 73.49%, H 12.33%, N 9.02%; found: C 73.09%, H 12.04%, N 8.93%.
¹H-NMR (CDCl₃), δ (ppm): 0.9 (t, 3H), 1.1-1.6 (m, 24H), 1.7 (m, 4H), 2.0 (m, 2H), 2.6 (t, 2H), 2.7 (m, 1H), 3.6 (m, 1 H).
¹³C-NMR (CDCl₃), δ (ppm): 14.04, 20.58, 21.17, 25.05, 25.97, 32.81, 32.84, 34.60, 46.74, 47.22, 48.20, 59.76, 81.69.

### Example 4: Synthesis of compound B' by reaction of NOR building block B with cyanuric chloride and ethanolamine:

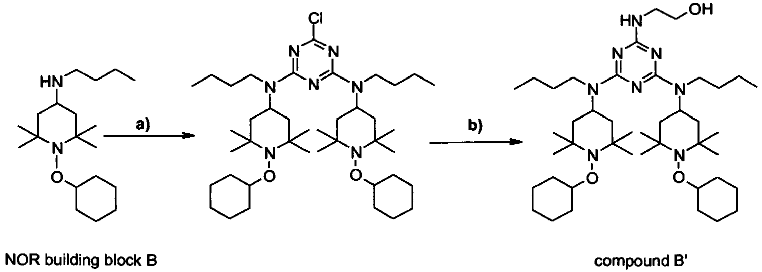

**a)** A solution of 6.2g (20mmol) NOR building block B in 10g cyclohexane is slowly added at 25°C to a stirred suspension of 1.9g (10mmol) cyanuric chloride in 8.4g cyclohexane. Stirring is continued for 30 minutes followed by the addition of 2.7g (20.4mmol) aqueous 30% NaOH solution. The mixture is heated to 70°C and stirred until the reaction is complete. The mixture is cooled down to 25°C, filtered, the aqueous phase split off and the organic phase washed with brine and concentrated on a rotary evaporator.
**b)** Excess ethanolamine (4g, 65mmol) is added and the solution heated to 110°C. Stirring is continued until the reaction is complete. The mixture is cooled down to 25°C, ethanolamine split off after addition of cyclohexane and the cyclohexane phase washed and evaporated to give a white powder.

Analysis required for C₄₃H₈₀N₈O₃ (757.17): C 68.21%, H 10.65%, N 14.80%; found: C 68.37%, H 10.60%, N 14.05%.

The as-prepared product exhibits higher quality compared to state-of-the-art material in terms of monomer content and transmission:

| compound B' | HPLC [Area%]^{a)} | Transmission [%]^{b)} |
|---|---|---|
| State of the art (Tinuvin® 152; CAS-no. 150686-79-6)) | 53 | 85.5 |
| prepared according to Example 4 (reactant NOR building block B not distilled) | 42 | 76.4 |
| prepared according to Example 4 (reactant NOR building block B distilled) | 80 | 88.6 |

| | | |
|---|---|---|
| ^{a)} Area of product (monomer) peak (retention time 28min) relative to sum of peaks; conditions: AAD-0004/2 with modified column (ZORBAX Extend C-18 column, 4.6mm x 250mm / 5µm, AGILENT No. 770450-902; column exhibiting enhanced stability at high pH); ^{b)} 425nm, 10% w/v solutions in m-xylene. | | |

### Example 5: Synthesis of compound C' by reaction of NOR building block B with cyanuric chloride and N,N'-bis(3-aminopropyl)ethylenediamine:

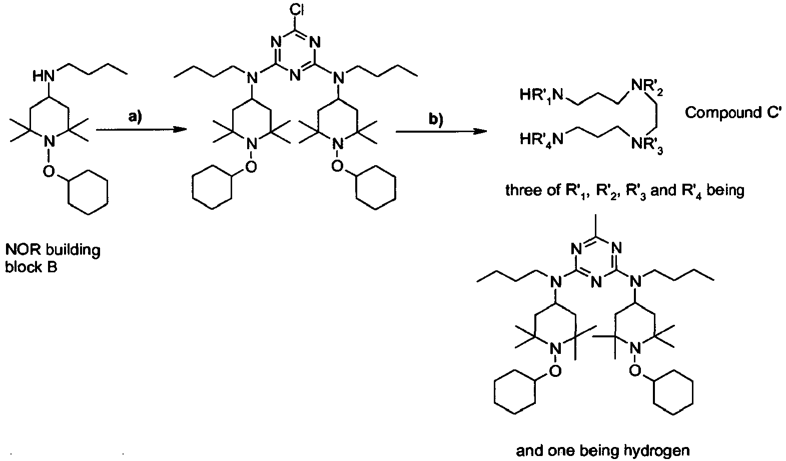

**a)** A solution of 6.5g NOR building block B in 10g cyclohexane is slowly added at 25°C to a stirred suspension of 1.9g (10mmol) cyanuric chloride in 10g cyclohexane. Stirring is continued for 30 minutes followed by the addition of 2.7g (20.4mmol) aqueous 30% NaOH solution. The mixture is heated to 70°C and stirred until the reaction is complete. The mixture is cooled down to 25°C, filtered, the aqueous phase split off and the organic phase washed with brine and concentrated on a rotary evaporator.
**b)** A mixture of 6g (8.2mmol) of the above crude product, 0.47g (2.7mmol) N,N'-bis(3-aminopropyl)ethylenediamine and 1.7g (8.5mmol) aqueous 20% NaOH solution is heated in a glass pressure bottle to 125°C during 17.5 hrs. The mixture is cooled down to 25°C, diluted with cyclohexane and the aqueous phase split off. The organic phase is brine washed and concentrated on a rotary evaporator. The crude oil is slowly added to boiling methanol, yielding a white precipitate. The suspension is treated with an Ultraturrax, filtered and the filtercake dried to yield the product as a white powder.

Analysis required for C₁₃₁H₂₄₁N₂₅O₆ (2262.51): C 69.54%, H 10.74%, N 15.48%; found: C 69.56%, H 10.60%, N 15.25%.

The as-prepared product exhibits higher quality compared to state-of-the-art material in terms of transmission:

| Compound C' | Transmission [%]^{b} | | |
|---|---|---|---|
| | 425nm | 450nm | 500nm |
| State of the art (Flamestab® NOR 116; CAS-no. 191680-81-6) | 68 | 75 | 84 |
| prepared according to Example 5 | 81 | 88 | 94 |

### Example 6: Preparation of NOR building block C in two steps from triacetonamine N-oxide:

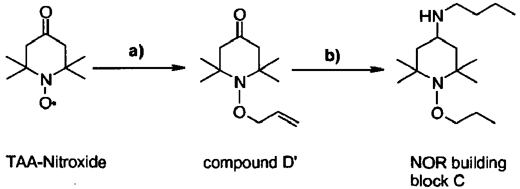

**a)** In a 300ml stainless steel autoclave are added 5.01g (29.45mmol) triacetone amine N-oxide, 198mg (0.9mmol) CuBr₂ and 286mg (0.9mmol) Bu₄NBr. The autoclave is sealed and 38.6g (920mmol) of propylene are added. The reaction is heated to 70°C (pressure ca 28 bar). When the temperature is reached, 7.6g (58.8mmol) of t-BuOOH (aqueous 70%) are added during 2.5 hours. The reaction is stirred for an additional 2 hours. The measured oxygen concentration of the gas phase is uncritical (<5%) throughout the reaction. Then the pressure in the autoclave is released. The autoclave is unloaded and rinsed with 50ml of dichloromethane. GLC analysis of the reaction mixture reveals about 90% conversion. The solvents are removed from the reaction mixture and the crude product (6.9g) purified by flash chromatography (silica gel, hexane / ethylacetate 3/1). Yield 4.1g (66%) of a white solid (mp 50 - 51°C; bp ca 80°C / 1 mbar).
   Analysis required for C₁₂H₂₁NO₂ (211.31): C 68.21%, H 10.02%, N 6.63%; found: C 68.76%, H 10.15%, N 6.55%.
   ¹H-NMR (400MHz, CDCl₃), δ (ppm): 1.18 (s, 6H), 1.31 (s, 6H), 2.22 (d, J = 12.8Hz, 2H), 2.57 (d, J = 12.8Hz, 2H), 4.38 (d x t, J = 5.6Hz / 1.2Hz, 2H), 5.17 (d x q, J = 10.6Hz / 1.6Hz, 1H), 5.30 (d x q, J = 17.4Hz / 1.6Hz, 1 H), 5.88 - 5.95 (m, 1 H).
   ¹³C-NMR (100MHz, CDCl₃), δ (ppm): 22.4 (2 CH₃), 32.4 (2 CH₃), 53.2 (2 CH₂), 62.9 (2 CN), 78.4 (OCH₂), 116.6 (CH₂), 133.3 (CH), 207.8 (CO).
   LC/MS (m/z): 212 (MH⁺)
**b)** A mixture of 86.7g (0.41 mol) compound D', 35.2g (0.476mol) butylamine and 0.8g 10% Pt on charcoal is hydrogenated over night at 80°C and 50 bar. Filtration and evaporation of volatiles yields 104.2g (93.9%) of a slightly yellow oil.
   Analysis required for C₁₆H₃₄N₂O (270.46): C 71.06%, H 12.67%, N 10.36%; found: C 70.86%, H 12.54%, N 10.49%.
   ¹H-NMR (400MHz, CDCl₃), δ (ppm): 0.93 (q, 6H), 1.17 (s, 6H), 1.19 (s, 6H), 1.2-1.31 (m, 2H), 1.32-1.37 (m, 2H), 1.41-1.47 (m, 2H), 1.51-1.56 (m, 2H), 1.71-1.74 (m, 2H), 2.59 (t, 2H), 2.73-2.78 (m, 1H), 3.69 (t, 2H).
   ¹³C(DEPT)-NMR (100MHz, CDCl₃), δ (ppm): 10.95 (CH₃), 14.03 (CH₃), 20.6 (CH₂), 21.0 (CH₃), 21.8 (CH₂), 32.8 (CH₂), 33.3 (CH₃), 46.8 (CH₂), 48.2 (CH), 59.8 (C), 78.4 (CH₂).

### Example 7: Preparation of NOR building block D in one step

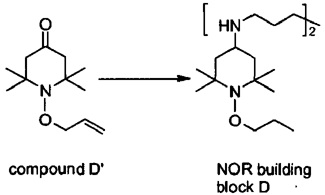

A mixture of 279g (1.32mol) compound D', 71.8g (0.6mol) 1,6-diaminohexane, 420ml ethanol and 1.2g 10% Pt on charcoal is hydrogenated over night at 100°C and 50 bar. The reaction mixture is filtered and volatiles evaporated to yield 315.5g (100%) of a slightly orange, viscous oil.

Analysis required for C₃₀H₆₂N₄O₂ (510.85): C 70.54%, H 12.23%, N 10.97%; found: C 70.47%, H 12.39%, N 10.94%.
¹H-NMR (400MHz, CDCl₃), δ (ppm): 0.95 (t, 6H), 1.15 (s, 12H), 1.18 (s, 12H), 1.20-1.26 (m, 4H), 1.34-1.36 (br m, 4H), 1.46-1.49 (m, 4H), 1.51-1.58 (m, 4H), 1.72-1.75 (m, 4H), 2.60 (t, 4H), 2.75-2.80 (m, 2H), 3.71 (t, 4H).
¹³C(DEPT)-NMR (100MHz, CDCl₃), δ (ppm): 10.95 (CH₃), 20.95 (CH₃), 21.96 (CH₂), 27.38 (CH₂), 30.57 (CH₂), 33.24 (CH₃), 46.63 (CH₂), 46.98 (CH₂), 48.14 (CH), 59.73 (C), 78.45 (CH₂).

### Example 8: Reaction of cyanuric chloride with NOR building blocks C and D

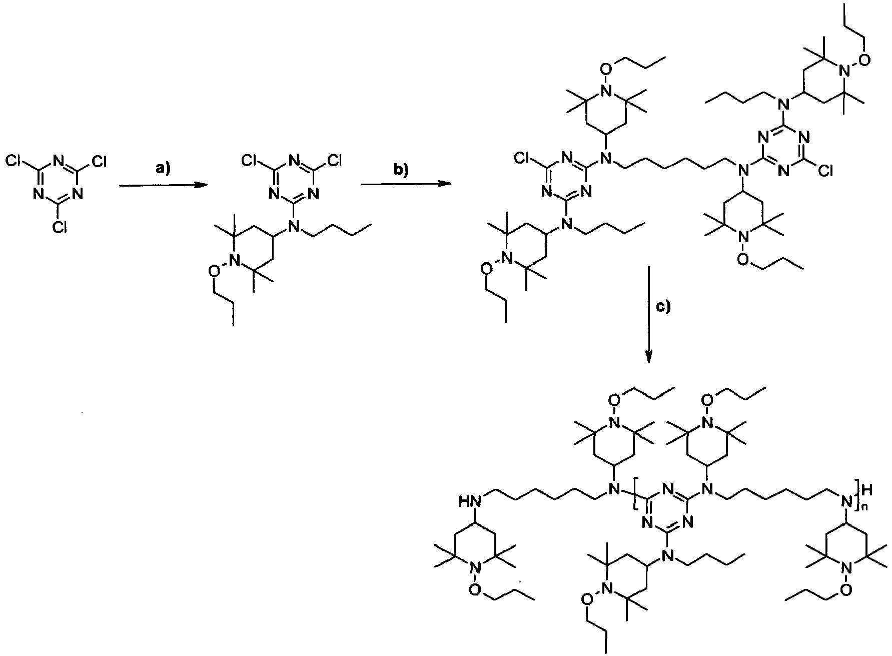

**a)** To a suspension of 24g (0.13mol) cyanuric chloride in 125ml xylene are slowly added at 5-10°C 35.2g (0.13mol) NOR building block C. The mixture is allowed to warm up to 40°C followed by the addition of 29g (0.145mol) NaOH (aqueous 20%). After stirring for one hour at 40°C, a sample is withdrawn and analyzed. GLC indicates >90% conversion. The structure is confirmed by NMR.
**b)** The aqueous phase is split off and the organic phase heated to 70°C followed by the slow addition of 33.2g (0.065mol) melted NOR building block D and 33g water. After addition of NaOH (aqueous 30%, 20g, 0.15mol) the mixture is brought to 80°C where it is left for one hour. The structure is confirmed by NMR.
**c)** The hot aqueous phase is split off. The organic phase is cooled down to 25°C and transferred into an autoclave. After addition of 66.4g (0.13mol) NOR building block D and 28.6g (0.143mol) NaOH (aqueous 20%) the autoclave is sealed and heated to 175°C where it is left for 4 hours. After cooling down to 25°C the autoclave is unloaded and the aqueous phase split off (at 80°C). The structure is confirmed by NMR. Mn / Mw (GPC) 1700 / 3300 - 1900 / 3800. Amount of residual NOR building block D ca 10% (area%).
**d)** Further reaction with 2-chloro-4,6-bis(dibutylamino)-s-triazine yields:

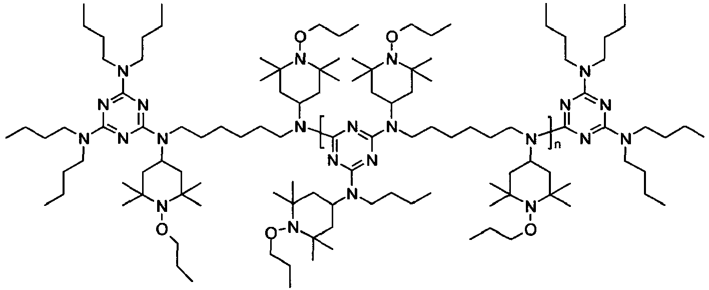

### Example 9: Preparation of compound D'

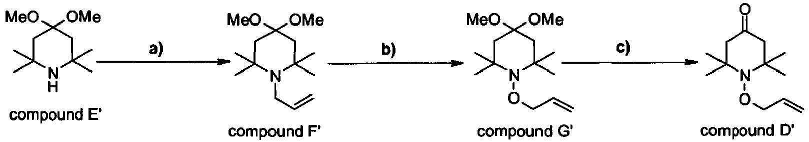

**a)** A mixture of 15.1g (75mmol) compound E' (synthesized according to EP748849, Rhone-Poulenc), 20g (150mmol) NaOH (aqueous 30%), 1.27g (3.7mmol) Bu₄NHSO₄ and 18.3g (151.3mmol) allylbromide is stirred at 90°C during 24 hours (95% conversion by GLC). The mixture is cooled down to 25°C followed by the addition of toluene (20ml). The aqueous phase is split off and the organic phase concentrated on a rotary evaporator. Distillation of the residue affords 8.1g (45%) of a slightly yellow oil.
   Analysis required for C₁₄H₂₇NO₂ (241.38): C 69.67%, H 11.27%, N 5.80%; found: C 69.62%, H 10.95%, N 5.77%.
   ¹H-NMR (300MHz, CDCl₃), δ (ppm): 1.10 (s, 12H), 1.72 (s, 4H), 3.18-3.21 (m, 2H), 3.19 (s, 6H), 4.94 (d x q, J = 10.2 Hz / 2Hz, 1H), 5.16 (d x q, J = 17.1Hz / 2Hz, 1H), 5.80-5.92 (m, 1H).
**b)** To a mixture of 23g (95mmol) compound F' and 19.28g (182mmol) Na₂CO₃ in 200ml toluene is added at -5°C 20.48g (105mmol) AcOOH (39% in AcOH) during 40 minutes. The mixture is stirred at 0°C (6 hours; 83% conversion by GLC) and then filtered. The filtrate is washed with NaOH 1M (3x20ml) and brine (3x20ml). The organic phase is dried (Na₂SO₄), filtered and the solvent evaporated. The residue is flash-filtrated over silicagel (hexane) to afford, after evaporation of the solvent, 15g (61%) of a yellow liquid.
   Analysis required for C₁₄H₂₇NO₃ (257.38): C 65.33%, H 10.57%, N 5.44%; found: C 65.48%, H 10.80%, N 5.33%.
   ¹H-NMR (400MHz, CDCl₃), δ (ppm): 1.10 (s, 6H), 1.27 (s, 6H), 1.59 (d, J = 13Hz, 2H), 1.94 (d, J = 13Hz, 2H), 3.17 (s, 6H), 4.30 (d x t, J = 5.2Hz / 1.6Hz, 2H), 5.14 (d x q, J = 10.4Hz / 1.6Hz, 1 H), 5.29 (d x q, J = 17.4Hz / 1.6Hz, 1 H), 5.86-5.96 (m, 1 H).
**c)** A solution of 1g (3.9mmol) compound G', 1g water and one drop (pasteur pipette) HCl (aqueous 32%) in 6ml THF is stirred at 25°C. After 4 hours (97% conversion by GLC) NaHCO₃ is added, the mixture filtrated and the filtrate concentrated on a rotary evaporator. The residue is extracted with hexane to afford, after evaporation of the solvent, 0.62g (75%) of a white solid.
   ¹H-NMR: same as in Example 6 a.

### Example 10: Preparation of compound D' using an oxidation methodology published by H. Adam et al., J. Org. Chem. 61, 1467-1472 (1996)

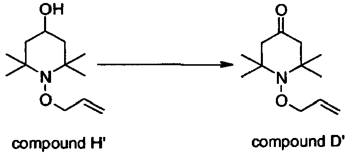

To a mixture of 5.05g (23.7mmol) compound H' (synthesized according to Ciba patent DE19907945), 1.03g (2.7mmol) Zr(OtBu)₄ and 9.5g activated molecular sieve (4A) in 45ml toluene are slowly added at 25°C 10.66g (47.3mmol) t-BuOOH (40% in cyclohexane). The mixture is stirred at 25°C for 24 hours (86% conversion by GLC) and then washed with saturated aqueous sodium potassium tartrate and brine. The aqueous phase is split off and the organic phase dried (Na₂SO₄). Evaporation of the solvent yields 3g of a slightly orange solid, which is analyzed by 400MHz ¹H-NMR adding 4,4'-di-tert-butylbiphenyl as internal standard. Yield calculated based on NO-C*H*₂CH=CH₂ (δ = 4.38ppm) 2.1g (42%).
¹H-NMR: same as in Example 6 a.

### Example 11: Preparation of compound G'

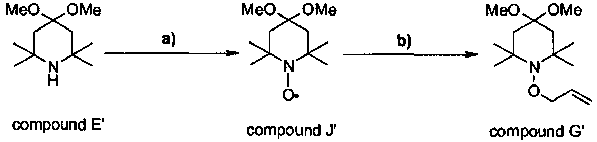

**a)** To a mixture of 2.1g (10mmol) compound E' and 0.16g (0.48mmol) Na₂WO₄x2H₂O in 10ml water are slowly added at 5°C 2.7g (24mmol) H₂O₂ (aqueous 30%). The mixture is stirred at 25°C until the starting material had disappeared (6 hours). Diethylether (20ml) is added and the aqueous phase saturated with K₂CO₃. The aqueous phase is split off and washed with diethylether. The organic phases are combined, the solvent evaporated and the residue dried on an oil pump to afford 2.15g (99%) of a red liquid.
   Analysis required for C₁₁H₂₂NO₃ (216.30): C 61.08%, H 10.25%, N 6.48%; found: C 61.03%, H 10.08%, N 6.39%.
**b)** Compound G' is synthesized in analogy to example 6a from 6.35g (29.4mmol) compound J', 38.6g (920 mmol) propylene, 0.328g (0.9 mmol) Bu₄NI and 7.6g (58.8mmol) t-BuOOH (aqueous 70%). GLC analysis of the reaction mixture reveals about 50% conversion. Non-reacted CG43-0819 is separated off by flash-chromatography (silica gel, hexane / ethylacetate 8 / 2) and the dried residue analyzed by 400MHz ¹H-NMR adding 4,4'-di-tert-butylbiphenyl as internal standard. Yield calculated based on NO-CH₂CH=CH₂ (δ = 4.30ppm) 1.5g (20%).
   ¹H-NMR: same as in Example 9 b. LC/MS (m/z): 258 (MH⁺)

### Example 12: Preparation of compound K' by oxidation of Chimassorb® 2020:

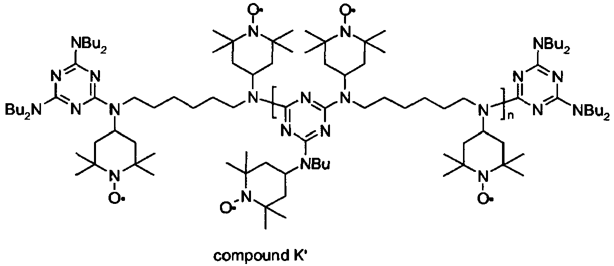

To a mixture of 20g Chimassorb® 2020 (commercially available from Ciba Specialty Chemicals; Mₙ by GPC: 2819 g/mol, ca. 3.5meq NH/g; CAS-no. 192268-64-7) and 35.5g (336.5mmol) Na₂CO₃ in 40ml of CH₂Cl₂ are slowly added at -5°C 26.6g (136.5mmol) of AcOOH (39% in AcOH). The mixture is kept stirable by concomitant, slow addition of a total of 90ml of water. The mixture is then stirred overnight at 20°C and the organic phase split off. The aqueous phase is extracted with CH₂Cl₂ and the combined organic phases washed with NaOH and brine, dried over MgSO₄ and the solvent evaporated to afford 18g of a red powder.
Analysis: found C 65.17%, H 10.00%, N 16.84%, O 6.64%.

### Example 13: Preparation of compound L' from compound K' via t-BuOOH hydrogen abstraction from propylene:

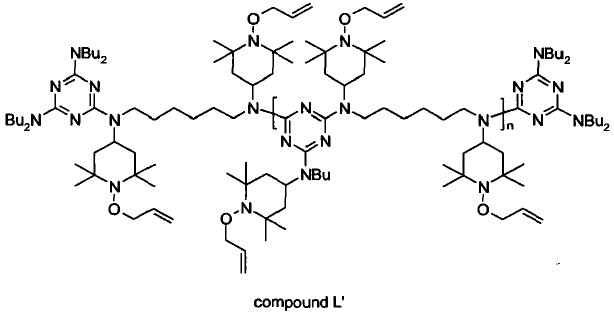

An autoclave is charged with 2.23g compound K' (ca 3.3meq NO/g), 0.081g (0.22mmol) Bu₄NI and 10ml chlororbenzene. The autoclave is sealed followed by the addition of 19.3g (458.6mmol) propylene. The system is then brought to 70°C (ca 22bar), whereafter 2.85g (22.1mmol) t-BuOOH (aqueous 70%) are slowly pumped in during 2 hours. The reaction mixture is held for another 30 minutes at 70°C and then cooled to 25°C (ca 10bar). Pressure is released and the autoclave uncharged. Volatiles are removed on a rotary evaporator and the residue dried, affording compound L' as yellowish powder.
¹H-NMR (300MHz, CDCl₃), δ (ppm, NO-CH₂CHCH₂ only): 4.3 (br s)

## Claims

1. A process for the preparation of a sterically hindered amine ether of the formula (100) 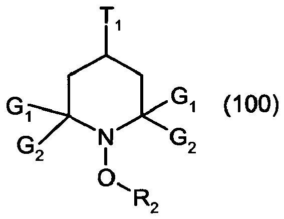 wherein
G₁ and G₂ are independently C₁-C₄alkyl;
R₂ is C₃-C₁₈alkyl or C₅-C₁₂cycloalkyl;
T₁ is hydroxy, -NT₂T₃, -OT₂₂, T₂₀ or a group of formula (102);
T₂ is hydrogen, C₅-C₁₂cycloalkyl or R₄₂; or T₂ is R₄₂ substituted by C₁-C₁₈alkoxy, aryl, hydroxy, carboxy, -CO-O-R₄₂, or -O-CO-R₄₂;
T₃ is hydrogen, C₅-C₁₂cycloalkyl, R₄₂, aryl, -Q-NHT₂ or -Q-NT₂T₂₁; or T₃ is R₄₂ substituted by C₁-C₁₈alkoxy, aryl, hydroxy, carboxy, -CO-O-R₄₂, or -O-CO-R₄₂; or T₃ is aryl substituted by C₁-C₁₈alkoxy, aryl, hydroxy, carboxy, -CO-O-R₄₂, -O-CO-R₄₂ or halogen;
or T₂ and T₃ form together C₄-C₁₁alkylene or C₄-C₁₁alkylene substituted by C₁-C₁₈alkoxy, aryl, hydroxy, carboxy, -CO-O-R₄₂, or -O-CO-R₄₂;
with the proviso that T₂ and T₃ are not benzyl;
R₄₂ is C₁-C₁₈alkyl;
Q is C₂-C₁₈alkylene, C₅-C₁₂cycloalkylene or phenylene;
T₂₂ is -(CO)-(C₁-C₁₆alkylene)_{0 or 1}-(CO)-O-T₂₁;
T₂₁ is 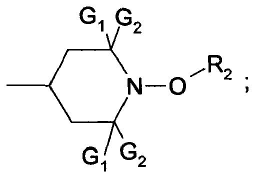
T₂₀ is 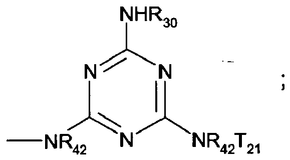
R₃₀ is R₄₂ or R₄₂ substituted by hydroxy; or R₃₀ is -(CH₂)ₙ-NT₂₃-(CH₂)ₚ-NT₂₃-(CH₂)ₙ-NHT₂₃ with one T₂₃ substituent being hydrogen and two T₂₃ substituents being 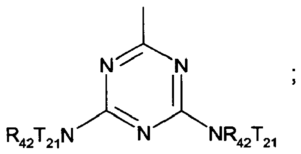
n is 1 to 4;
p is 1 to 3;
the group of formula (102) is 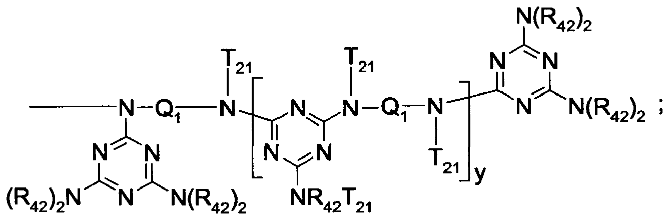
y is 2 to 20;
which comprises transforming a compound of formula (101), 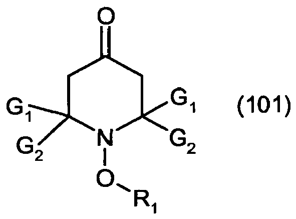 wherein
R₁ is C₃-C₁₈alkenyl or C₅-C₁₂cycloalkenyl,
in one reaction step in the presence of hydrogen and a catalyst into a compound of formula (100) wherein T₁ is hydroxy or -NT₂T₃;
whereby for obtaining compounds with T₁ = -NT₂T₃ the transformation is performed in the presence of an amine of formula HNT₂T₃₀;
T₃₀ is hydrogen, C₅-C₁₂cycloalkyl, R₄₂, aryl or -Q-NHT₂; or T₃₀ is R₄₂ substituted by C₁-C₁₈alkoxy, aryl, hydroxy, carboxy, -CO-O-R₄₂, or -O-CO-R₄₂; or T₃₀ is aryl substituted by C₁-C₁₈alkoxy, aryl, hydroxy, carboxy, -CO-O-R₄₂, -O-CO-R₄₂ or halogen;
or T₂ and T₃₀ form together C₄-C₁₁alkylene or C₄-C₁₁alkylene substituted by C₁-C₁₈alkoxy, aryl, hydroxy, carboxy, -CO-O-R₄₂, or -O-CO-R₄₂;
with the proviso that T₃₀ is not benzyl;
and for obtaining a compound of formula (100) with T₁ = -OT₂₂, reacting a compound of formula (100) with T₁ = hydroxy with an HOOC-(C₁-C₁₆alkylene)_{0 or 1}-COOH or a halide thereof or a methyl ester thereof;
for obtaining a compound of formula (100) with T₁ = T₂₀, and R₃₀ = R₄₂ substituted by hydroxy, reacting a compound of formula (100) with T₁ = -NT₂T₃, T₂ = H, T₃ = R₄₂ with a cyanuric halide to yield a compound of formula (103), which is subsequently reacted with R₄₂NH₂ or hydroxy-substituted R₄₂NH₂; 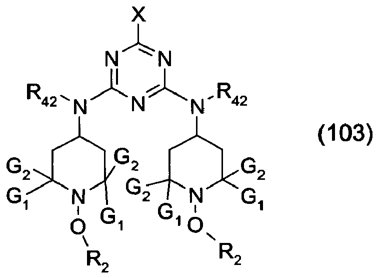 wherein
X is halogen;
for obtaining a compound of formula (100) with T₁ = T₂₀ and R₃₀ = -(CH₂)ₙ-NT₂₃-(CH₂)ₚ-NT₂₃-(CH₂)ₙ-NHT₂₃,
a compound of formula (103) is reacted with H₂N-(CH₂)ₙ-NH-(CH₂)ₚ-NH-(CH₂)ₙ-NH₂; and
for obtaining a compound of formula (100) with T₁ = group of formula (102), reacting a compound of formula (100) with T₁ = -NT₂T₃, T₂ = H, T₃ = R₄₂, with a cyanuric halide to yield a compound of formula (104),
which is subsequently reacted with a compound of formula (100) with T₁ = -NT₂T₃, T₂ = H, T₃ = -Q-NHT₂₁, to yield a compound of formula (105),
which is subsequently reacted with a compound of formula (100) with T₁ = -NT₂T₃, T₂ = H, T₃ = -Q-NHT₂₁, to yield a compound of formula (106),
which is subsequently reacted with a compound 2-X-4,6-bis((R₄₂)₂amino)-s-triazine.

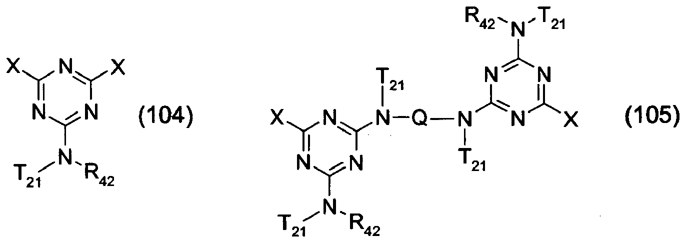

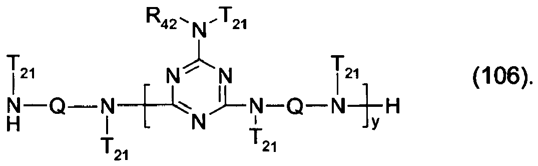

2. A process according to claim 1, wherein the catalyst is Ru, Pt or Pd on charcoal or Raney-Ni.

3. A process according to claim 1 or 2, wherein the transformation is carried out at a temperature of 35 - 120°C and a hydrogen pressure of 6 -100 bar.

4. A process according to claim 3, wherein the temperature is 45 - 110°C and the hydrogen pressure is 8 - 60 bar.

5. A process according to claim 1, wherein
R₂ is C₃-C₁₀alkyl or C₅-C₇cycloalkyl;
T₂ is hydrogen;
T₃ is R₄₂, -Q-NHT₂ or -Q-NT₂T₂₁;
R₄₂ is C₁-C₈alkyl;
Q is C₂-C₈alkylene;
T₂₂ is -(CO)-C₄-C₁₀alkylene-(CO)-O-T₂₁;
n is 2 to 4;
y is 2 to 10
R₁ is C₃-C₁₀alkenyl or C₅-C₇cycloalkenyl and
X is chlorine, bromine or iodine.

6. A process according to claim 1, wherein the compound of formula (101) is obtained by reacting a compound of formula (200) with a C₃-C₁₈alkene or C₅-C₁₂cycloalkene.

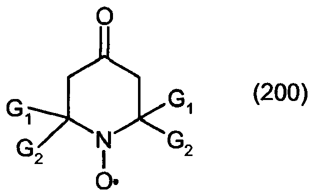

7. A process according to claim 6, wherein the compound of formula (200) is obtained by oxidizing a compound of formula (201).

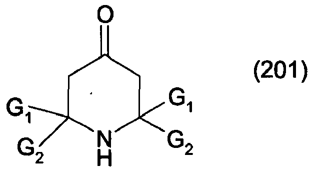

8. A process according to claims 1, 6 or 7, which comprises the conversion of a compound of formula (201) to a compound of formula (100) without the isolation of the intermediate products.

9. A process according to claims 1 or 6, which comprises the conversion of a compound of formula (200) to a compound of formula (100) without the isolation of the intermediate products.

10. A process according to claim 1, wherein the compound of formula (101) with R₁ being the group 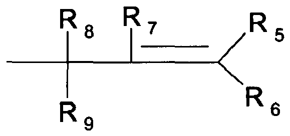 wherein
R₅, R₆, R₇, R₈ and R₉, independently of each other, are H, C₁-C₈alkyl, C₂-C₈alkenyl; and R₇ and R₈ together may also form a chemical bond;
is obtained by reacting a compound of formula (202) with a compound of formula (203), 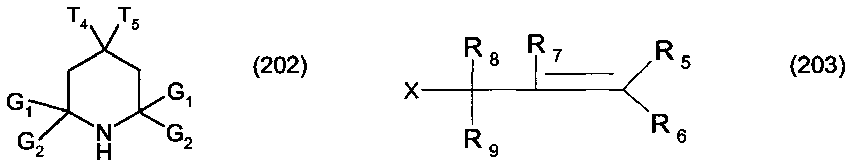 wherein T₄ and T₅ are independently C₁-C₁₈alkoxy; or T₄ is hydroxy and T₅ is hydrogen; X is halogen;
affording a compound of formula (204); 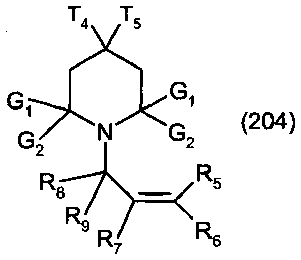 oxidizing the compound of formula (204) in the presence of oxygen, peroxides, permanganates or chlorates affords a compound of formula (205); and 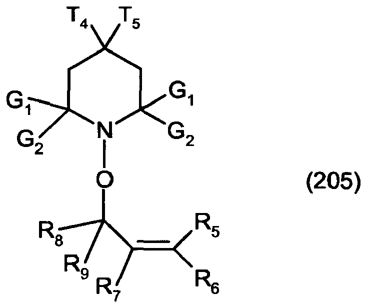 deacetalising the compound of formula (205) with T₄ and T₅ being independently C₁-C₁₈alkoxy or oxidizing the compound of formula (205) with T₄ = hydroxy and T₅ = hydrogen.

11. A process according to claim 1, 6, 7 or 10, wherein G₁ and G₂ are methyl.

## Patentansprüche

1. Verfahren zum Herstellen eines sterisch gehinderten Aminethers der Formel (100), 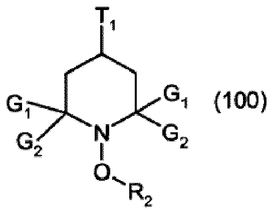 wobei
G₁ und G₂ unabhängig C₁-C₄Alkyl sind;
R₂ C₃-C₁₈Alkyl oder C₅-C₁₂Cycloalkyl ist;
T₁ Hydroxy, -NT₂T₃, -OT₂₂, T₂₀ oder eine Gruppe der Formel (102) ist;
T₂ Wasserstoff, C₅-C₁₂Cycloalkyl oder R₄₂ ist; oder T₂ R₄₂ substituiert mit C₁-C₁₈Alkoxy, Aryl, Hydroxy, Carboxy, -CO-O-R₄₂ oder -O-CO-R₄₂ ist;
T₃ Wasserstoff, C₅-C₁₂Cycloalkyl, R₄₂, Aryl, -Q-NHT₂ oder -Q-NT₂T₂₁ ist; oder T₃ R₄₂ substituiert mit C₁-C₁₈Alkoxy, Aryl, Hydroxy, Carboxy, -CO-O-R₄₂ oder -O-CO-R₄₂ ist; oder T₃ Aryl substituiert mit C₁-C₁₈Alkoxy, Aryl, Hydroxy, Carboxy, -CO-O-R₄₂, -O-CO-R₄₂ oder Halogen ist;
oder T₂ und T₃ zusammen C₄-C₁₁Alkylen oder C₄-C₁₁Alkylen substituiert mit C₁-C₁₈Alkoxy, Aryl, Hydroxy, Carboxy, -CO-O-R₄₂ oder -O-CO-R₄₂ bilden;
mit der Maßgabe, dass T₂ und T₃ nicht Benzyl sind;
R₄₂ C₁-C₁₈Alkyl ist;
Q C₂-C₁₈Alkylen, C₅-C₁₂Cycloalkylen oder Phenylen ist;
T₂₂-(CO)-(C₁-C₁₆Alkylen)_{0 oder 1}-(CO)-O-T₂₁ ist;
T21 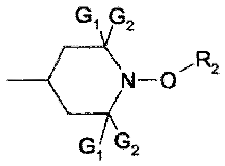 ist;
T₂₀ 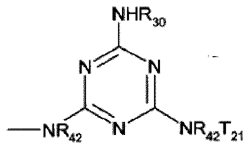 ist;
R₃₀ R₄₂ oder R₄₂ substituiert mit Hydroxy ist; oder
R₃₀ -(CH₂)ₙ-NT₂₃-(CH₂)ₚ-NT₂₃-(CH₂)ₙ-NHT₂₃ ist, wobei ein T₂₃-Substituent Wasserstoff ist und zwei T₂₃-Substituenten 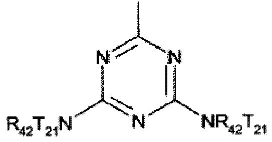 sind;
n 1 bis 4 ist;
p 1 bis 3 ist;
die Gruppe der Formel (102) 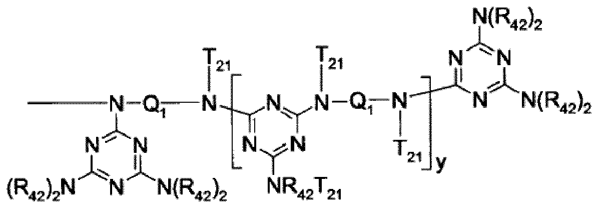 ist;
y 2 bis 20 ist;
umfassend Umwandeln einer Verbindung der Formel (101), 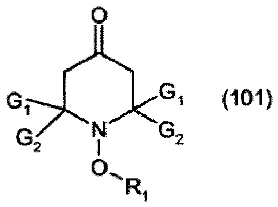 wobei
R₁ C₃-C₁₈Alkenyl oder C₅-C₁₂Cycloalkenyl ist,
in einem Reaktionsschritt in Gegenwart von Wasserstoff und eines Katalysators zu einer Verbindung der Formel (100), wobei T₁ Hydroxy oder -NT₂T₃ ist;
wobei zum Erhalten von Verbindungen mit T₁ = -NT₂T₃ die Umwandlung in Gegenwart eines Amins der Formel HNT₂T₃₀ durchgeführt wird;
T₃₀ Wasserstoff, C₅-C₁₂Cycloalkyl, R₄₂, Aryl oder -Q-NHT₂ ist; oder T₃₀ R₄₂ substituiert mit C₁-C₁₈Alkoxy, Aryl, Hydroxy, Carboxy, -CO-O-R₄₂ oder -O-CO-R₄₂ ist; oder T₃₀ Aryl substituiert mit C₁-C₁₈Alkoxy, Aryl, Hydroxy, Carboxy, -CO-O-R₄₂, -O-CO-R₄₂ oder Halogen ist;
oder T₂ und T₃₀ zusammen C₄-C₁₁Alkylen oder C₄-C₁₁Alkylen substituiert mit C₁-C₁₈Alkoxy, Aryl, Hydroxy, Carboxy, -CO-O-R₄₂ oder -O-CO-R₄₂ bilden;
mit der Maßgabe, dass T₃₀ nicht Benzyl ist;
und zum Erhalten einer Verbindung der Formel (100) mit T₁ = -OT₂₂ Umsetzen einer Verbindung der Formel (100) mit T₁ = Hydroxy mit einem HOOC-(C₁-C₁₆Alkylen)_{0 oder 1}-COOH oder einem Halogenid davon oder einem Methylester davon;
zum Erhalten einer Verbindung der Formel (100) mit T₁ = T₂₀ und R₃₀ = R₄₂ substituiert mit Hydroxy Umsetzen einer Verbindung der Formel (100) mit T₁ = -NT₂T₃, T₂ = H, T₃ = R₄₂ mit einem Cyanursäurehalogenid, um eine Verbindung der Formel (103) zu ergeben, die anschließend mit R₄₂NH₂ oder Hydroxy-substituiertem R₄₂NH₂ umgesetzt wird; 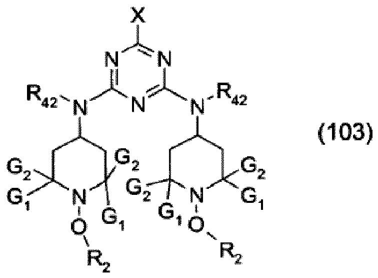 wobei
X Halogen ist;
zum Erhalten einer Verbindung der Formel (100) mit T₁ = T₂₀ und R₃₀ = -(CH₂)ₙ-NT₂₃-(CH₂)ₚ-NT₂₃-(CH₂)ₙ-NHT₂₃ Umsetzen einer Verbindung der Formel (103) mit H₂N-(CH₂)ₙ-NH-(CH₂)ₚ-NH-(CH₂)ₙ-NH₂; und
zum Erhalten einer Verbindung der Formel (100) mit T₁ = Gruppe der Formel (102) Umsetzen einer Verbindung der Formel (100) mit T₁ = -NT₂T₃, T₂ = H, T₃ = R₄₂ mit einem Cyanursäurehalogenid, um eine Verbindung der Formel (104) zu ergeben,
die anschließend mit einer Verbindung der Formel (100) mit T₁ = -NT₂T₃, T₂ = H, T₃ = -Q-NHT₂₁ umgesetzt wird, um eine Verbindung der Formel (105) zu ergeben,
die anschließend mit einer Verbindung der Formel (100) mit T₁ = -NT₂T₃, T₂ = H, T₃ = -Q-NHT₂₁ umgesetzt wird, um eine Verbindung der Formel (106) zu ergeben,
die anschließend mit einer Verbindung 2-X-4,6-Bis((R₄₂)₂Amino)-s-triazin umgesetzt wird,

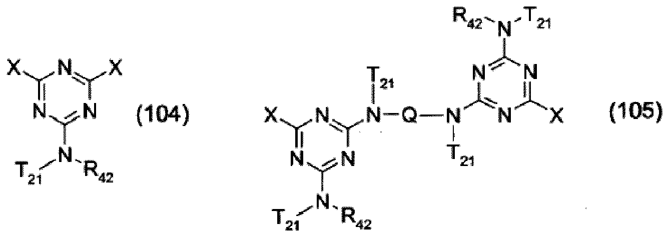

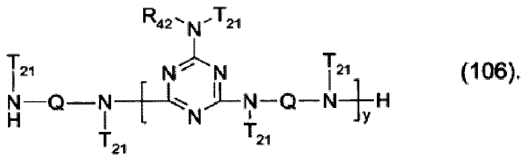

2. Verfahren gemäß Anspruch 1, wobei der Katalysator Ru, Pt oder Pd auf Kohle oder Raney-Ni ist.

3. Verfahren gemäß Anspruch 1 oder 2, wobei die Umwandlung bei einer Temperatur von 35 - 120 °C und einem Wasserstoffdruck von 6 - 100 bar durchgeführt wird.

4. Verfahren gemäß Anspruch 3, wobei die Temperatur 45 - 110 °C beträgt und der Wasserstoffdruck 8 - 60 bar beträgt.

5. Verfahren gemäß Anspruch 1, wobei
R₂ C₃-C₁₀Alkyl oder C₅-C₇Cycloalkyl ist;
T₂ Wasserstoff ist;
T₃ R₄₂, -Q-NHT₂ oder -Q-NT₂T₂₁ ist;
R₄₂ C₁-C₈Alkyl ist;
Q C₂-C₈Alkylen ist; T₂₂ -(CO)-C₄-C₁₀Alkylen-(CO)-O-T₂₁ ist;
n 2 bis 4 ist;
y 2 bis 10 ist;
R₁ C₃-C₁₀Alkenyl oder C₅-C₇Cycloalkenyl ist und
X Chlor, Brom oder Iod ist.

6. Verfahren gemäß Anspruch 1, wobei die Verbindung der Formel (101) durch Umsetzen einer Verbindung der Formel (200) mit einem C₃-C₁₈Alken oder C₅-C₁₂Cycloalken erhalten wird,

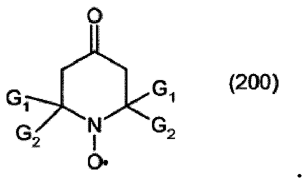

7. Verfahren gemäß Anspruch 6, wobei die Verbindung der Formel (200) durch Oxidieren einer Verbindung der Formel (201) erhalten wird,

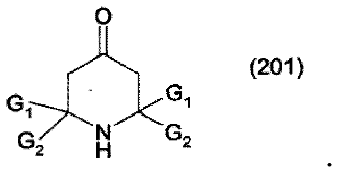

8. Verfahren gemäß Ansprüchen 1, 6 oder 7, umfassend die Umwandlung einer Verbindung der Formel (201) zu einer Verbindung der Formel (100) ohne Isolieren der Intermediatprodukte.

9. Verfahren gemäß Ansprüchen 1 oder 6, umfassend die Umwandlung einer Verbindung der Formel (200) zu einer Verbindung der Formel (100) ohne Isolieren der Intermediatprodukte.

10. Verfahren gemäß Anspruch 1, wobei die Verbindung der Formel (101), bei der R₁ die Gruppe 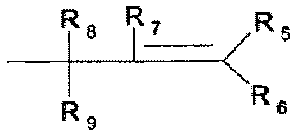 ist, wobei
R₅, R₆, R₇, R₈ und R₉ unabhängig voneinander H, C₁-C₈Alkyl, C₂-C₈Alkenyl sind; und R₇ und R₈ zusammen auch eine chemische Bindung bilden können;
erhalten wird durch Umsetzen einer Verbindung der Formel (202) mit einer Verbindung der Formel (203), 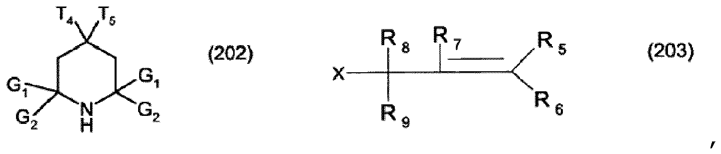
wobei T₄ und T₅ unabhängig C₁-C₁₈Alkoxy sind; oder T₄ Hydroxy ist und T₅ Wasserstoff ist; X Halogen ist;
um eine Verbindung der Formel (204) zu ergeben; 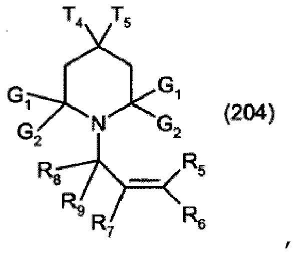
Oxidieren der Verbindung der Formel (204) in Gegenwart von Sauerstoff, Peroxiden, Permanganaten oder Chloraten, um eine Verbindung der Formel (205) zu liefern; und 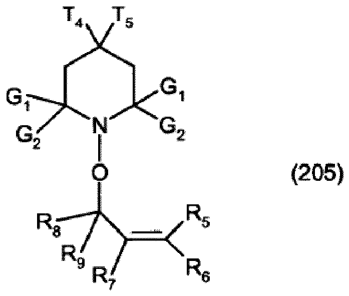
Deacetalisieren der Verbindung der Formel (205), wobei T₄ und T₅ unabhängig C₁-C₁₈Alkoxy sind, oder Oxidieren der Verbindung der Formel (205) mit T₄ = Hydroxy und T5 = Wasserstoff.

11. Verfahren gemäß Anspruch 1, 6, 7 oder 10, wobei G₁ und G₂ Methyl sind.

## Revendications

1. Procédé de préparation d'un éther d'amine stériquement encombrée de formule (100) 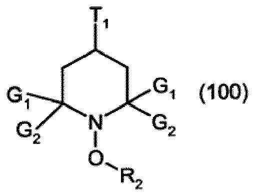 dans laquelle
G₁ et G₂ sont indépendamment C₁-C₄alkyle ;
R₂ est C₃-C₁₈alkyle ou C₅-C₁₂cycloalkyle ;
T₁ est hydroxy, -NT₂T₃, -OT₂₂, T₂₀ ou un groupement de formule (102) ;
T₂ est hydrogène, C₅-C₁₂cycloalkyle ou R₄₂ ; ou T₂ est R₄₂ substitué par C₁-C₁₈alcoxy, aryle, hydroxy, carboxy, -CO-O-R₄₂, ou -O-CO-R₄₂ ;
T₃ est hydrogène, C₅-C₁₂cycloalkyle, R₄₂, aryle, -Q-NHT₂ ou -Q-NT₂T₂₁ ; ou T₃ est R₄₂ substitué par C₁-C₁₈alcoxy, aryle, hydroxy, carboxy, -CO-O-R₄₂, ou -O-CO-R₄₂ ; ou T₃ est aryle substitué par C₁-C₁₈alcoxy, aryle, hydroxy, carboxy, -CO-O-R₄₂, -O-CO-R₄₂ ou halogène ;
ou T₂ et T₃ forment ensemble C₄-C₁₁alkylène ou C₄-C₁₁alkylène substitué par C₁-C₁₈alcoxy, aryle, hydroxy, carboxy, -CO-O-R₄₂, ou -O-CO-R₄₂ ;
à condition que T₂ et T₃ ne soient pas benzyle ;
R₄₂ est C₁-C₁₈alkyle ;
Q est C₂-C₁₈alkylène, C₅-C₁₂cycloalkylène ou phénylène ;
T₂₂ est -(CO)-(C₁-C₁₆alkylène)_{0 ou 1}-(CO)-O-T₂₁ ;
T₂₁ est 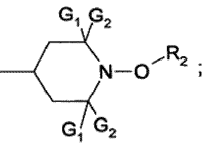
T₂₀ est 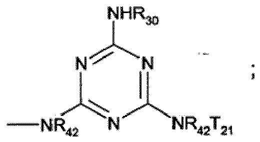
R₃₀ est R₄₂ ou R₄₂ substitué par hydroxy ; ou R₃₀ est -(CH₂)ₙ-NT₂₃-(CH₂)ₚ-NT₂₃-(CH₂)ₙ-NHT₂₃ avec un substituant T₂₃ qui est hydrogène et deux substituants T₂₃ qui sont 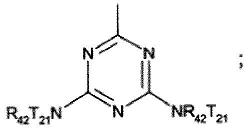
n va de 1 à 4 ;
p va de 1 à 3 ;
le groupement de formule (102) est 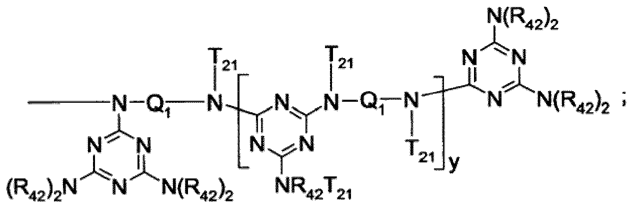
y va de 2 à 20 ;
comprenant la transformation d'un composé de formule (101), 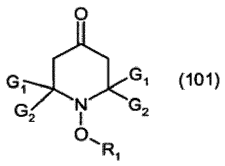 dans laquelle
R₁ est C₃-C₁₈alcényle ou C₅-C₁₂cycloalcényle,
en une seule étape de réaction en présence d'hydrogène et d'un catalyseur en un composé de formule (100) dans laquelle T₁ est hydroxy ou -NT₂T₃ ;
où pour l'obtention de composés avec T₁= -NT₂T₃, la transformation est effectuée en présence d'une amine de formule HNT₂T₃₀ ;
T₃₀ est hydrogène, C₅-C₁₂cycloalkyle, R₄₂, aryle ou -Q-NHT₂ ; ou T₃₀ est R₄₂ substitué par C₁-C₁₈alcoxy, aryle, hydroxy, carboxy, -CO-O-R₄₂, ou -O-CO-R₄₂ ; ou T₃₀ est aryle substitué par C₁-C₁₈alcoxy, aryle, hydroxy, carboxy, -CO-O-R₄₂, -O-CO-R₄₂ ou halogène ;
ou T₂ et T₃₀ forment ensemble C₄-C₁₁alkylène ou C₄-C₁₁alkylène substitué par C₁-C₁₈alcoxy, aryle, hydroxy, carboxy, -CO-O-R₄₂, ou -O-CO-R₄₂ ;
à condition que T₃₀ ne soit pas benzyle ;
et afin d'obtenir un composé de formule (100) avec T₁= -OT₂₂, la réaction d'un composé de formule (100) avec T₁= hydroxy avec un HOOC-(C₁-C₁₆alkylène)_{0 ou 1}-COOH ou un halogénure de celui-ci ou un méthylester de celui-ci ;
afin d'obtenir un composé de formule (100) avec T₁= T₂₀, et R₃₀= R₄₂ substitué par hydroxy, la réaction d'un composé de formule (100) avec T₁= -NT₂T₃, T₂= H, T₃= R₄₂ avec un halogénure cyanurique pour conduire à un composé de formule (103), qui est ensuite réagi avec R₄₂NH₂ ou R₄₂NH₂ substitué par hydroxy ; 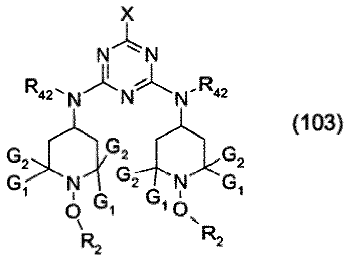 dans laquelle
X est halogène ;
afin d'obtenir un composé de formule (100) avec T₁= T₂₀ et R₃₀= -(CH₂)ₙ-NT₂₃-(CH₂)ₚ-NT₂₃-(CH₂)ₙ-NHT₂₃,
un composé de formule (103) est réagi avec H₂N-(CH₂)ₙ-NH-(CH₂)ₚ-NH-(CH₂)ₙ-NH₂ ; et
afin d'obtenir un composé de formule (100) avec T₁= groupement de formule (102), la réaction d'un composé de formule (100) avec T₁= -NT₂T₃, T₂= H, T₃= R₄₂, avec un halogénure cyanurique pour conduire à un composé de formule (104),
qui est ensuite réagi avec un composé de formule (100) avec T₁= -NT₂T₃, T₂= H, T₃= -Q-NHT₂₁, pour conduire à un composé de formule (105),
qui est ensuite réagi avec un composé de formule (100) avec T₁= -NT₂T₃, T₂= H, T₃= -Q-NHT₂₁, pour conduire à un composé de formule (106),
qui est ensuite réagi avec un composé de 2-X-4,6-bis ((R₄₂)₂-amino) -s-triazine

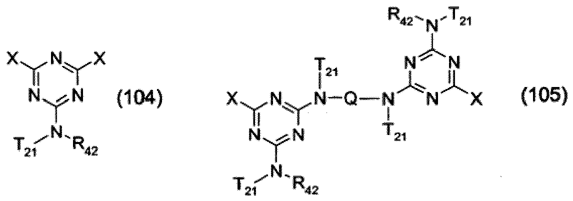

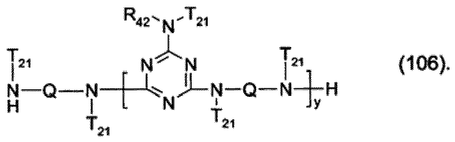

2. Procédé selon la revendication 1, dans lequel le catalyseur est Ru, Pt ou Pd sur charbon ou Raney-Ni.

3. Procédé selon la revendication 1 ou 2, dans lequel la transformation est effectuée à une température de 35-120°C et une pression d'hydrogène de 6-100 bars.

4. Procédé selon la revendication 3, dans lequel la température est de 45-110°C et la pression d'hydrogène est de 8-60 bars.

5. Procédé selon la revendication 1, dans lequel
R₂ est C₃-C₁₀alkyle ou C₅-C₇cycloalkyle ;
T₂ est hydrogène ;
T₃ est R₄₂, -Q-NHT₂ ou -Q-NT₂T₂₁ ;
R₄₂ est C₁-C₈alkyle ;
Q est C₂-C₈alkylène ;
T₂₂ est -(CO)-C₄-C₁₀alkylène-(CO)-O-T₂₁ ;
n va de 2 à 4 ;
y va de 2 à 10 ;
R₁ est C₃-C₁₀alcényle ou C₅-C₇cycloalcényle et
X est chlore, brome ou iode.

6. Procédé selon la revendication 1, dans lequel le composé de formule (101) est obtenu par la réaction d'un composé de formule (200) avec un C₃-C₁₈alcéne ou C₅-C₁₂cycloalcéne

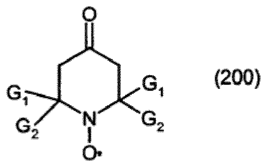

7. Procédé selon la revendication 6, dans lequel le composé de formule (200) est obtenu par l'oxydation d'un composé de formule (201)

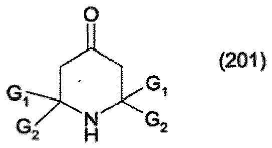

8. Procédé selon les revendications 1, 6 ou 7, comprenant la conversion d'un composé de formule (201) en un composé de formule (100) sans isolement des produits intermédiaires.

9. Procédé selon les revendications 1 ou 6, comprenant la conversion d'un composé de formule (200) en un composé de formule (100) sans isolement des produits intermédiaires.

10. Procédé selon la revendication 1, dans lequel le composé de formule (101) dans laquelle R₁ est le groupement 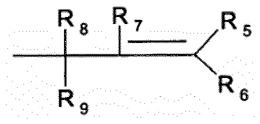 où
R₅, R₆, R₇, R₈ et R₉, indépendamment les uns des autres, sont H, C₁-C₈alkyle, C₂-C₈alcényle ; et R₇ et R₈ peuvent également former ensemble une liaison chimique ;
est obtenu par la réaction d'un composé de formule (202) avec un composé de formule (203), 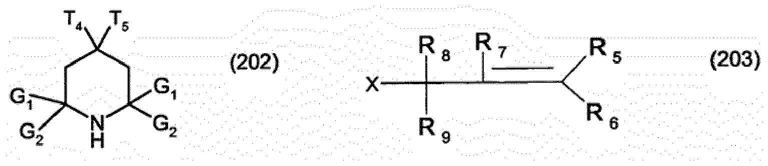 dans laquelle
T₄ et T₅ sont indépendamment C₁-C₁₈alcoxy ; ou T₄ est hydroxy et T₅ est hydrogène ; X est halogène ;
conduisant à un composé de formule (204) ; 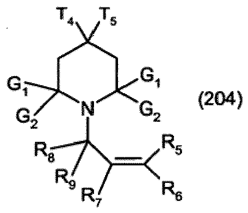
l'oxydation du composé de formule (204) en présence d'oxygène, de peroxydes, de permanganates ou de chlorates conduit à un composé de formule (205) ; et 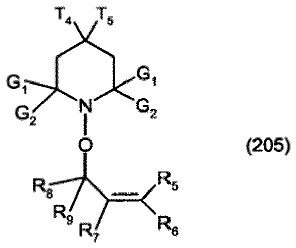
la désacétalisation du composé de formule (205) dans laquelle T₄ et T₅ sont indépendamment C₁-C₁₈alcoxy ou l'oxydation du composé de formule (205) dans laquelle T₄= hydroxy et T₅= hydrogène.

11. Procédé selon la revendication 1, 6, 7 ou 10, dans lequel G₁ et G₂ sont méthyle.
